# EUROPÄISCHE PATENTSCHRIFT

(11) **EP 2 054 380 B1**
(45) Veröffentlichungstag und Bekanntmachung des Hinweises auf die Patenterteilung: **11.09.2013**
(21) Anmeldenummer: 07819274.7
(22) Anmeldetag: 24.10.2007
(51) Int. Cl.: C07C 311/33, C07D 213/56, C07D 213/89, C07K 5/06, C07D 233/64, A61K 31/44, A61P 7/02

(54) **TRYPSINARTIGE SERINPROTEASE-HEMMSTOFFE, IHRE HERSTELLUNG UND VERWENDUNG**
TRYPSIN-LIKE SERINE PROTEASE INHIBITORS, AND THEIR PREPARATION AND USE
INHIBITEURS DE SERINE PROTEASE DE TYPE TRYPSINE, LEUR FABRICATION ET LEUR UTILISATION

(30) Priorität: 24.10.2006 DE 102006050672
(43) Veröffentlichungstag der Anmeldung: 06.05.2009
(60) Teilanmeldung: 12004643.8
(73) Patentinhaber: The Medicines Company (Leipzig) GmbH, 04103 Leipzig (DE)
(72) Erfinder: STEINMETZER, Torsten, 07743 Jena (DE); SCHWEINITZ, Andrea, 07749 Jena (DE); STÜRZEBECHER, Jörg, 99094 Erfurt (DE); STEINMETZER, Peter, 04179 Leipzig (DE); SÖFFING, Anett, 99423 Weimar (DE); VAN DE LOCHT, Andreas, 80469 München (DE); NICKLISCH, Silke, 04105 Leipzig (DE); REICHELT, Claudia, 04299 Leipzig (DE); LUDWIG, Friedrich-Alexander, 04275 Leipzig (DE); SCHULZE, Alexander, 04924 Bad Liebenwerda (DE); DAGHISCH, Mohammed, 04109 Leipzig (DE); HEINICKE, Jochen, 04229 Leipzig (DE)
(74) Vertreter: Bösl, Raphael Konrad
(86) Internationale Anmeldenummer: PCT/EP2007/009220
(87) Internationale Veröffentlichungsnummer: WO 2008/049595

(56) Entgegenhaltungen:
- WO-A-03/076457
- WO-A-2004/062657
- WO-A1-00/58346
- WO-A1-2005/026198
- US-A- 5 602 253

## Beschreibung

Die Erfindung betrifft Hemmstoffe trypsinartiger Serinproteasen der allgemeinen Formel 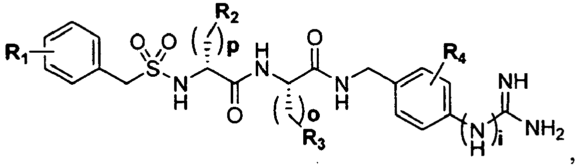 die neben Plasmin auch Plasmakallikrein hemmen, sowie deren Herstellung und Verwendung als Arzneimittel vorzugsweise zur Behandlung von Blutverlust, insbesondere bei hyperfibrinolytischen Zuständen, bei Organtransplantationen oder herzchirurgischen Eingriffen vor allem mit cardiopulmonalem Bypass, oder als Bestandteil eines Fibrinklebers.

Inhibitoren für Plasmin und Plasmakallikrein (PK) sind bereits bekannt. Plasmin ist eine trypsinartige Serinprotease und spaltet zahlreiche Substrate C-terminal der basischen Aminosäuren Arginin oder Lysin. Plasmin wird aus dem Zymogen Plasminogen durch katalytische Wirkung der Plasminogenaktivatoren Urokinase oder tPA gebildet. Zu den Plasminsubstraten gehören verschiedene Proteine der extrazellulären Matrix und Basalmembran, beispielsweise Fibronectin, Laminin, Type IV-Kollagen oder Fibrin, aber auch zahlreiche Zymogene, wie Proformen der Matrix-Metalloproteasen oder des Plasminogenaktivators Urokinase. Im Blut ist Plasmin vor allem für die Fibrinolyse verantwortlich, indem es Fibrin in lösliche Produkte spaltet.

Zu den endogenen Plasmininhibitoren gehören α2-Macroglobulin und das Serpin α2-Antiplasmin. Unter bestimmten pathologischen Bedingungen kann es zu einer spontanen Aktivierung der Fibrinolyse kommen. Im Falle einer derartigen Hyperplasminämie wird nicht nur das wundverschließende Fibrin abgebaut, sondern es werden auch gerinnungshemmende Fibrinogenabbauprodukte gebildet. Dadurch können schwere Blutstillungsstörungen auftreten. Als Antifibrinolytika werden in der Klinik synthetische Aminocarbonsäuren, wie ε-Aminocapronsäure, p-Aminomethylbenzoesäure oder Tranexaminsäure (trans-4-Aminomethylcyclo-hexancarbonsäure) verwendet. Diese Verbindungen blockieren die Bindung des Zymogens Plasminogen am Fibrin und hemmen dadurch dessen Aktivierung zu Plasmin. Deshalb sind diese Verbindungen keine direkten Hemmstoffe des Plasmins und können bereits gebildetes Plasmin nicht inhibieren. Als weiteres Antifibrinolytikum wird Aprotinin (Trasylol^{®}, Bayer AG, Leverkusen) eingesetzt, ein Polypeptid aus 58 Aminosäuren, das aus Rinderlunge gewonnen wird. Aprotinin inhibiert Plasmin mit einer Hemmkonstante von 1 nM, ist aber relativ unspezifisch und hemmt auch wirksam Trypsin (Kᵢ = 0,1 nM) und Plasmakallikrein (Kᵢ = 30 nM). Aprotinin inhibiert noch weitere Enzyme, allerdings mit verminderter Wirksamkeit.

Eine Hauptanwendung des Aprotinins dient der Reduktion von Blutverlust, insbesondere bei herzchirurgischen Eingriffen mit cardiopulmonalem Bypass (CPB), wodurch der Bedarf an perioperativen Bluttransfusionen deutlich reduziert wird (Sodha et al., 2006). Zusätzlich wird Aprotinin auch bei anderen Operationen, beispielsweise bei Organtransplantationen, zur Hemmung von Blutverlust eingesetzt oder als Zusatz in Fibrinkleber verwendet.

Die Verwendung von Aprotinin hat mehrere Nachteile. Da es aus Rinderorganen isoliert wird, besteht prinzipiell die Gefahr pathogener Kontaminationen und allergischen Reaktionen. Das Risiko eines anaphylaktischen Schocks ist bei erstmaliger Gabe von Aprotinin relativ gering (< 0,1 %), erhöht sich bei wiederholter Gabe innerhalb von 200 Tagen allerdings auf 4-5 %.

Vor kurzem wurde berichtet, dass die Gabe von Aprotinin im direkten Vergleich zu ε-Aminocapronsäure oder Tranexaminsäure eine erhöhte Anzahl an Nebenwirkungen induziert (Mangano et al., 2006). Durch die Gabe von Aprotinin kam es zu einer Verdopplung der Anzahl von Nierenschädigungen, wodurch eine Dialyse erforderlich wurde. Ebenso erhöhte sich durch die Gabe von Aprotinin das Herzinfarkt- und Hirnschlagrisiko im Vergleich zu den Kontrollgruppen.

Bisher sind erst wenige synthetische Inhibitoren des Plasmins bekannt. Von Sanders und Seto (1999) wurden relativ schwach wirksame 4-Heterocyclohexanon-Derivate mit Hemmkonstanten ≥ 50 µM für Plasmin beschrieben. Xue und Seto (2005) berichten von peptidischen Cyclohexanon-Derivaten mit IC₅₀-Werten ≥ 2 µM, deren Weiterentwicklung ist aber nicht bekannt. Von Okada und Tsuda wurden verschiedene Derivate mit einem 4-Aminomethylcyclohexanoyl-Rest beschrieben, die Plasmin mit IC₅₀-Werten ≥ 0,1 µM inhibieren, eine klinische Anwendung dieser Inhibitoren ist nicht bekannt (Okada et al., 2000; Tsuda et al., 2001).

In zahlreichen Publikationen zur Entwicklung von Hemmstoffen der Gerinnungsproteasen als Antithrombotika wurden Hemmkonstanten für Plasmin publiziert, wobei in diesen Fällen allerdings das Ziel in einer möglichst schwachen Plasminhemmung bestand. In keiner dieser Arbeiten wurde eine mögliche Verwendung dieser Verbindungen zur Reduktion von Blutverlust bei herzchirurgischen Eingriffen genannt. So hemmt beispielsweise der Thrombininhibitor Melagatran Plasmin mit einem Kᵢ-Wert von 0,7 µM während die strukturell eng verwandte Verbindungen H317/86 eine Hemmkonstante von 0,22 µM für Plasmin besitzt (Gustafsson et al., 1998). Beide Verbindungen hemmen allerdings die Protease Thrombin deutlich stärker mit Kᵢ-Werten ≤ 2 nM, wodurch es durch die Gabe von Melagatran zu einer starken Gerinnungshemmung kommt.

Wie einleitend beschrieben, hemmt Aprotinin neben Plasmin zusätzlich Plasmakallikrein (PK). PK ist eine multifunktionelle, trypsinartige Serinprotease, für die mehrere physiologische Substrate bekannt sind. So kann PK durch proteolytische Spaltung das vasoaktive Peptid Bradykinin aus hochmolekularem Kininogen freisetzen und die Zymogene Gerinnungsfaktor XII, Pro-Urokinase, Plasminogen und Pro-MMP 3 aktivieren. Deshalb wird angenommen, dass das PK/Kinin-System eine wichtige Rolle bei verschiedenen Krankheitsbildern besitzt, so z.B. bei thromboembolischen Situationen, der disseminierten intravasalen Gerinnung, septischem Schock, Allergien, dem Postgastrektomiesyndrom, Arthritis und ARDS (adult respiratory distress syndrome) (Tada et al., 2001).

Demzufolge hemmt Aprotinin über seine inhibierende Wirkung auf PK die Freisetzung des Peptidhormons Bradykinin. Bradykinin bewirkt über die Aktivierung des Bradykinin-B2-Rezeptors verschiedene Effekte. Durch die Bradykinin-induzierte Freisetzung von tPA, NO und Prostacyclin aus Endothelzellen (siehe Übersichtsarbeit Schmaier, 2002) werden die Fibrinolyse, der Blutdruck und das Entzündungsgeschehen beeinflusst. Es wird diskutiert, dass durch Hemmung der Bradykinin-Freisetzung systemische Entzündungsvorgänge, die als Nebenwirkung bei Operationen auftreten können, reduziert werden.

Als PK-Inhibitoren wurden verschiede Bis-benzamidine wie Pentamidin und verwandte Verbindungen, sowie Ester von ω-Amino- und ω-Guanidinoalkylcarbonsäuren mit mikromolaren Kᵢ-Werten beschrieben (Asghar et al., 1976; Muramatu und Fuji, 1971; Muramatu und Fuji, 1972; Ohno et al., 1980; Muramatu et al., 1982; Satoh et al., 1985; Teno et al., 1991).

Die ersten selektiven kompetitiven Inhibitoren, die sich vom Arginin bzw. Phenylalanin ableiten, wurde von Okamoto et al., (1988) entwickelt und hemmen PK mit Kᵢ₋Werten um 1 µM. Von der Gruppe um Okada wurden mehrere Arbeiten zur Entwicklung kompetitiver PK-Inhibitoren veröffentlicht, wobei die wirksamsten Verbindungen, die sich vom trans-4-Amino-methylcyclohexancarbonyl-Phe-4-Carboxymethylanilid ableiten, Hemmkonstanten um 0,5 µM besitzen (Okada et al., 1999; Okada et al., 2000, Tsuda et al., 2001). Gemeinsam ist den genannten PK-Inhibitoren ihr relativ hoher Kᵢ-Wert. In US 6,472,393 wurden potente PK-Inhibitoren mit Hemmkonstanten um 1 nM beschrieben, die ein 4-Amidinoanilin als P1 Rest besitzen. PK-Hemmstoffe wurden auch in US 5,602,253 beschrieben. In US 2006/0148901 wurden PK-Hemmstoffe beschrieben, deren Hemmwirkung auf Plasmin aber relativ gering ist, wodurch sich diese Hemmstoffe von den Inhibitoren unterscheiden, die in der vorliegenden Anmeldung beschrieben werden.

In WO 2005/026198 A1 werden basisch-substituierte Benzylaminanaloga als Inhibitoren des Gerinnungsfaktors Xa, ihre Herstellung und Verwendung zur Therapie und Prophylaxe von kardiovaskulären Erkrankungen und thromboembolischen Erzeugnissen beschrieben.

In WO 2004/062657 A1 wird die Verwendung von acyliertem 4-Amidino- oder 4-Guanidinobenzylamin gemäß der allgemeinen Formel (I) P4-P3-P2-P1 (I) beschrieben, wobei P4 eine einfach oder mehrfach substituierte oder unsubstituierte Benzylsulfonylgruppe ist, P3 eine einfach oder mehrfach substituierte oder unsubstituierte, natürliche oder unnatürliche α-Amino- oder α-Iminosäure in der D-Konfiguration ist, P2 eine einfach oder mehrfach substituierte oder unsubstituierte, natürliche oder unnatürliche α-Amino- oder α-Iminosäure in der L-Konfiguration ist, und P 1 eine einfach oder mehrfach substituierte oder unsubstituierte 4-Amidino- oder 4-Guanidino-benzylamingruppe ist, zur Inhibierung von Plasmakallikrein (PK), Faktor XIa und Faktor IIa, insbesondere zur Verhinderung der Gerinnungsaktivierung an künstlichen Oberflächen und zur systemischen Gabe als Antikoagulanzien/Antithrombotika, vor allem zur Verhinderung der Gerinnungsaktivierung an künstlichen Oberflächen zur Vorbeugung thromboembolischer Ereignisse.

WO 03/076457 A1 offenbart Amidino- und Guanidinoderivate als Hemmstoffe des Gerinnungsfaktors Xa und deren Verwendung zur Therapie, Prophylaxe und Diagnose von kardiovaskularen Erkrankungen und Thromboembolishen Ereignissen.

In WO 00/58346 A1 werden N-Sulfonyl-Dipeptidderivate, deren Herstellung und deren therapeutische Verwendung beschrieben.

Der Erfindung liegt daher die Aufgabe zu Grunde, für therapeutische Anwendungen geeignete niedermolekulare Wirkstoffe bereitzustellen, die insbesondere Plasmin und Plasmakallikrein reversibel und kompetitiv mit hoher Aktivität und Spezifität inhibieren und daher zur Blutstillung bei verschiedenen Anwendungen geeignet sind, beispielsweise bei herzchirurgischen Eingriffen mit CPB, bei Organstransplantationen oder anderen Operationen. Ein weiterer Vorteil dieser Verbindungen besteht darin, dass durch ihre Wirkung als Hemmstoff des Plasmakallikreins zusätzlich die Kininfreisetzung vermindert wird und dadurch Kinin-vermittelte enzündliche Reaktionen unterdrückt werden können. Durch die gehemmte Kininfreisetzung wird wiederum die Kinin-induzierte Freisetzung von tPA aus Endothelzellen unterdrückt. wodurch über diesen Mechanismus die Fibrinolyse herabreguliert werden kann. Ein weiterer Vorteil dieser Verbindungen ist trotz Selektivität eine gewisse Hemmwirkung dieser Verbindungen auf FXa und/oder Thrombin, wodurch zusätzlich thrombotische Komplikationen bei der Anwendung dieser Verbindungen reduziert werden sollen.

Überraschenderweise wurde nun gefunden, dass durch die Kombination zweier sterisch anspruchsvoller und/oder hydrophober Reste R₂ und R₃ gemäß Formel 1, vorzugsweise substituierte oder unsubstituierte Aromaten, Inhibitoren mit starken Hemmkonstanten gegenüber Plasmin und Plasmakallikrein erhalten werden konnten. Vergleichbar gute Effekte konnten auch mit Substanzen erhalten werden, die an R₂ nichtaromatische und R₃ basisch substituierte Phenylreste tragen.

Gegenstand der vorliegenden Erfindung sind daher Verbindungen der allgemeinen Formel (I) 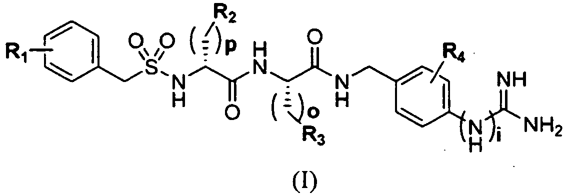 mit
R₁, gegebenenfalls einfach oder mehrfach vorhanden und unabhängig voneinander ein COOR₅-Rest, mit R₅ gleich Wasserstoff oder einer verzweigten oder linearen Niederalkylgruppe mit 1-6 Kohlenstoffatomen, vorzugsweise Methyl oder Ethyl, insbesondere Methyl, ein verzweigter oder linearer Aminoalkyl-Rest mit 1-6 Kohlenstoffatomen, vorzugsweise Methyl, ein Halogen- oder Pseudohalogen-Rest, vorzugsweise Chlor oder eine Cyanogruppe, oder ein Polyethylenglykol-Rest der Formel (II) oder (III)

CH₃-O-(CH₂-CH₂-O-)ₙ-CH₂-CH₂-(C=O)-NH-CH₂- (II)

CH₃-O-(CH₂-CH₂-O-)ₙ-CH₂-CH₂-NH-(C=O)-CH₂-CH₂-(C=O)-NH-CH₂- (III)

wobei n in der Regel so definiert ist, dass die genannten Polyethylenglykolreste ein durchschnittliches Molekulargewicht von 10000 Da, 5000 Da, 3400 Da, 2000 Da, 1000 Da oder 750 Da aufweisen. Üblicherweise ist n eine ganze Zahl zwischen ca. 18 bis ca. 250, insbesondere ca. 18, ca. 25, ca. 50, ca. 85, ca. 125 oder ca. 250.
R₂ ein unsubstituierter oder substituierter, aromatischer oder nichtaromatischer Cyclus oder Bicylus mit 5-13 Kohlenstoffatomen oder aromatischer Heterocyclus mit 4-5 Kohlenstoffatomen und einem Stickstoffatom, Stickstoffoxid, Sauerstoffatom oder Schwefelatom ist, worin die Substitution ein Halogenrest, vorzugsweise Chlor oder Fluor, insbesondere Chlor, ein gegebenenfalls mit Fluor substituierter, verzweigter oder linearer Alkyl-Rest mit 1-6 Kohlenstoffatomen, vorzugsweise Methyl oder tertiäres Butyl, ein gegebenenfalls mit Fluor substituierter, verzweigter oder linearer Alkyloxy-Rest mit 1-6 Kohlenstoffatomen, vorzugsweise Methyl, ein Hydroxy-Rest oder ein Cyano-Rest ist; oder ein Rest der Struktur: 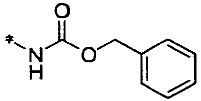 ist;
R₃ ausgewählt ist aus 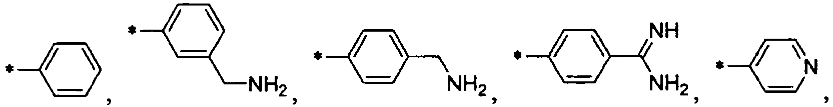 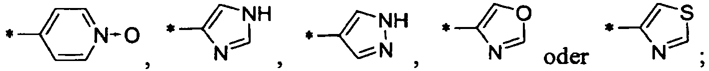 R₄ gegebenenfalls ein einfach oder mehrfach vorhandener Halogen-Rest, vorzugsweise Fluor;
o = 1, oder 2 insbesondere 1;
p = 0, 1, 2, 3 oder 4, insbesondere 3; und
i = 0 oder 1, insbesondere 0;
sowie deren racemische Mischungen und Salze mit organischen oder anorganischen Säuren.

Die experimentellen Ergebnisse haben gezeigt, dass die Plasmin- und die Plasmakallikreinhemmung besonders gut bei Verbindungen mit cyclischen Strukturen an R₂ und R₃ und insbesondere mit einem aromatischen Kohlenstoffcyclus an R₂ und R₃ ist. Weiterhin konnte gezeigt werden, dass durch geeignete Wahl der Substituenten zusätzlich eine gute Faktor Xa- und/oder Thrombinhemmung bei Verbindungen mit einem aromatischen Kohlenstoffcyclus an R₂ und R₃ erreicht werden konnte.

Die experimentellen Ergebnisse haben auch gezeigt, dass eine deutliche Verminderung der Hemmung von Thrombin erreicht wird, wenn R₁ eine 3-COOH-Gruppe darstellt. In einer bevorzugten Ausführungsform ist daher R₁ einfach und in meta- oder para-Stellung vorhanden, vorzugsweise ist R₁ ein COOH-Rest, insbesondere ist R₁ einfach vorhanden und ausgewählt aus Wasserstoff, einer 4-COOH-Gruppe oder insbesondere einer 3-COOH-Gruppe. Eine weitere Verminderung der Hemmung von Thrombin konnte dadurch erreicht werden, dass R₄ ein Fluoratom, insbesondere in ortho-Stellung, darstellt.

Eine weitere bevorzugte Ausführungsform der vorliegenden Erfindung betrifft Verbindungen, bei denen R₂ ein substituierter oder unsubstituierter, aromatischer Cyclus oder Bicylus mit 6-13 Kohlenstoffatomen oder Heterocyclus mit 5 Kohlenstoffatomen und einem Stickstoffatom ist.

Die Substitution an R₂ kann im allgemeinen ein Halogenrest, vorzugsweise Chlor oder Fluor, insbesondere Chlor, ein gegebenenfalls mit Fluor substituierter, verzweigter oder linearer Alkyl-Rest mit 1-6 Kohlenstoffatomen, vorzugsweise Methyl oder tertiäres Butyl, ein gegebenenfalls mit Fluor substituierter, verzweigter oder linearer Alkyloxy-Rest mit 1-6 Kohlenstoffatomen, vorzugsweise Methyl, ein Hydroxy-Rest oder ein Cyano-Rest sein.

In einer alternativen Ausführungsform kann auch R₂ ein nichtaromatischer Cyclus mit 6 Kohlenstoffatomen sein.

Als besonders geeignete Verbindungen haben sich Verbindungen der Formel (I) erwiesen, bei denen R₃ ausgewählt ist aus

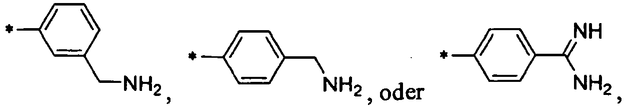

Vor allem hat sich eine Verbindung gemäß Formel (I) mit i = 0 und ohne R₄ mit folgenden Resten als besonders geeignet erwiesen.

| Verbindung Nr. | R₁ | R₂ | p | R₃ | o |
|---|---|---|---|---|---|
| 3 | 3-COOH | 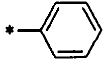 | 3 | 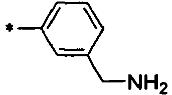 | 1 |

Die Salze der erfindungsgemäßen Verbindungen sind im allgemeinen gebildet aus Salzsäure, HBr, Essigsäure, Trifluoressigsäure Toluensulfonsäure oder anderen geeigneten Säuren.

Im Speziellen sind Verbindungen geeignet, bei denen R₂ ausgewählt ist aus folgenden Resten: 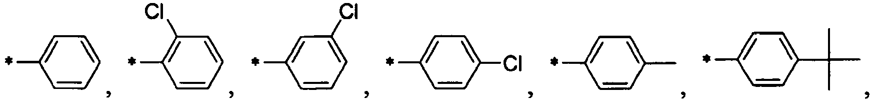 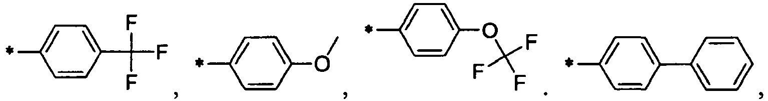 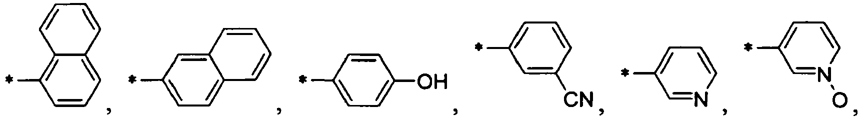 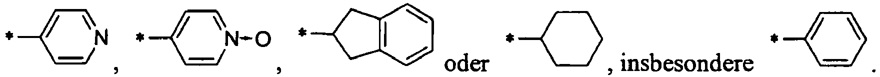 und/oder bei denen R₃ ausgewählt ist aus 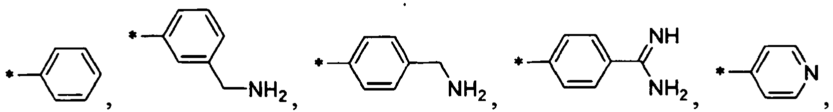 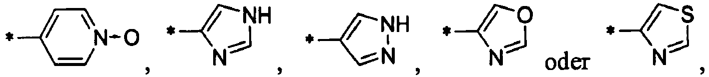 insbesondere

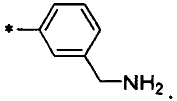

Beispiele von derartigen Verbindungen sind Verbindungen der Formel (I), die folgendermaßen definiert sind:

| Verbindung Nr. | R₁ | R₂ | p | R₃ | o | i | R₄ |
|---|---|---|---|---|---|---|---|
| 1 | H | 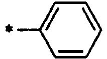 | 3 | 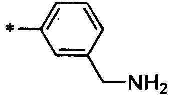 | 1 | 0 | - |
| 2 | 4-COOH | 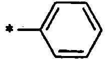 | 3 | 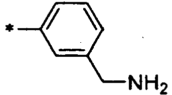 | 1 | 0 | - |
| 3 | 3-COOH | 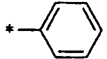 | 3 | 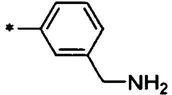 | 1 | 0 | - |
| 4 | 3-COOH | 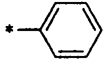 | 3 | 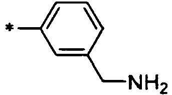 | 1 | 1 | - |
| 5 | 3-COOH | 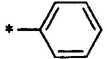 | 3 | 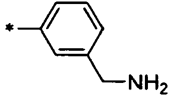 | 1 | 0 | 2-F |
| 6 | H | 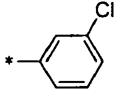 | 3 | 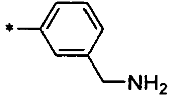 | 1 | 0 | - |
| 7 | H | 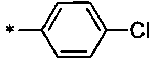 | 3 | 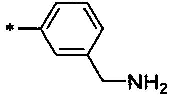 | 1 | 0 | - |
| 8 | H | 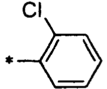 | 3 | 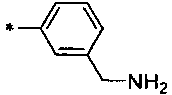 | 1 | 0 | - |
| 9 | H | 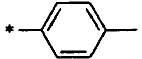 | 3 | 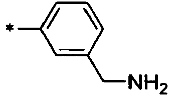 | 1 | 0 | - |
| 10 | H | 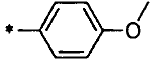 | 3 | 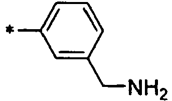 | 1 | 0 | - |
| 11 | H | 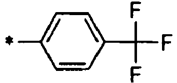 | 3 | 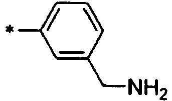 | 1 | 0 | - |
| 12 | H | 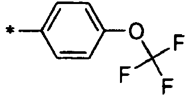 | 3 | 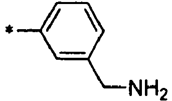 | 1 | 0 | - |
| 13 | H | 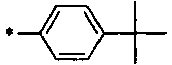 | 1 | 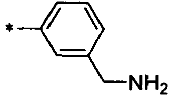 | 1 | 0 | - |
| 14 | H | 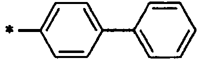 | 1 | 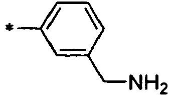 | 1 | 0 | - |
| 15 | H | 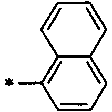 | 1 | 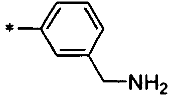 | 1 | 0 | - |
| 16 | 4-COOH | 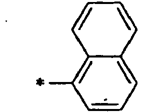 | 1 | 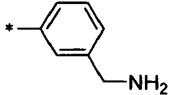 | 1 | 0 | - |
| 17 | H | 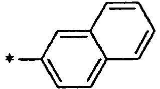 | 1 | 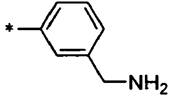 | 1 | 0 | - |
| 18 | 3-COOH | 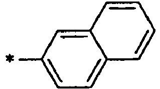 | 1 | 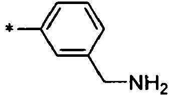 | 1 | 0 | - |
| 19 | 4-COOH | 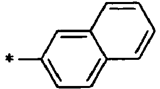 | 1 | 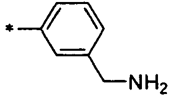 | 1 | 0 | - |
| 20 | H | 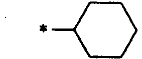 | 1 | 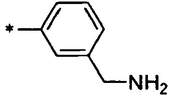 | 1 | 0 | - |
| 21 | H | 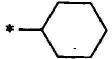 | 3 | 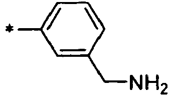 | 1 | 0 | - |
| 22 | H | 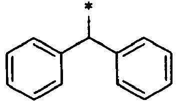 | 0 | 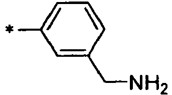 | 1 | 0 | - |
| 23 | 3-COOH | 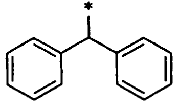 | 0 | 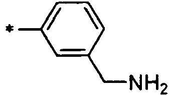 | 1 | 0 | - |
| 24 | H | 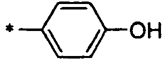 | 2 | 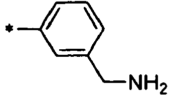 | 1 | 0 | - |
| 25 | 3-COOH | 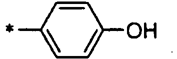 | 2 | 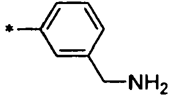 | 1 | 0 | - |
| 26 | H | 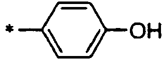 | 2 | 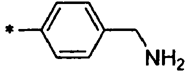 | 2 | 0 | - |
| 27 | H | 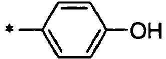 | 2 | 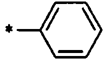 | 2 | 0 | - |
| 28 | H | 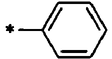 | 3 | 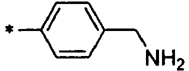 | 1 | 0 | - |
| 29 | 4-COOH | 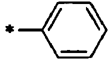 | 3 | 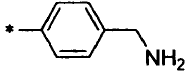 | 1 | 0 | - |
| 30 | 3-COOH | 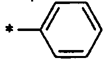 | 3 | 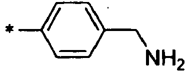 | 1 | 0 | - |
| 31 | H | 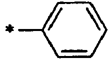 | 3 | 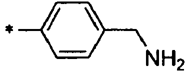 | 2 | 0 | - |
| 32 | H | 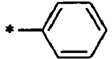 | 3 | 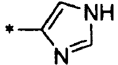 | 1 | 0 | - |
| 33 | 3-COOH | 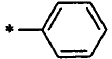 | 3 | 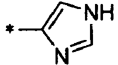 | 1 | 0 | - |
| 34 | H | 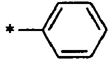 | 3 | 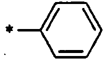 | 2 | 0 | - |
| 35 | 4-COOH | 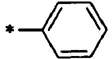 | 3 | 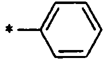 | 2 | 0 | - |
| 36 | 3-COOH | 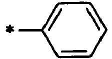 | 3 | 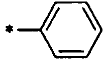 | 2 | 0 | - |
| 37 | H | 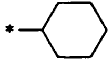 | 1 | 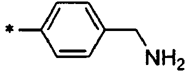 | 2 | 0 | - |
| 38 | H | 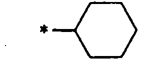 | 1 | 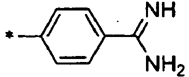 | 2 | 0 | - |
| 39 | H | 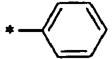 | 3 | 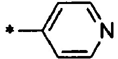 | 2 | 0 | - |
| 40 | 3-COOH | 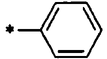 | 3 | 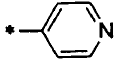 | 2 | 0 | - |
| 41 | H | 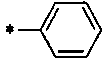 | 3 | 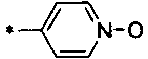 | 2 | 0 | - |
| 42 | 3-COOH | 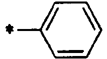 | 3 | 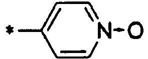 | 2 | 0 | - |
| 43 | H | 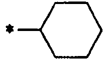 | 1 | 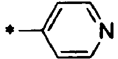 | 2 | 0 | - |
| 44 | H | 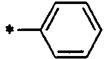 | 2 | 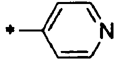 | 2 | 0 | - |
| 45 | H | 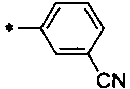 | 1 | 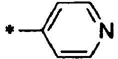 | 2 | 0 | - |
| 46 | H | 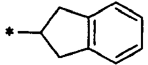 | 0 | 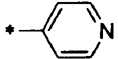 | 2 | 0 | - |
| 47 | H | 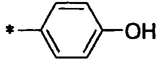 | 2 | 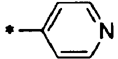 | 2 | 0 | - |
| 49 | H | 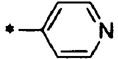 | 3 | 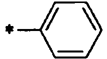 | 2 | 0 | - |
| 50 | 3-COOH | 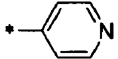 | 3 | 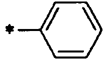 | 2 | 0 | - |
| 51 | H | 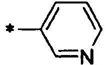 | 3 | 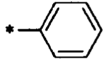 | 2 | 0 | - |
| 52 | 3-COOH | 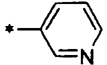 | 3 | 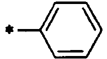 | 2 | 0 | - |
| 53 | H | 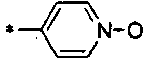 | 3 | 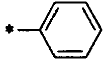 | 2 | 0 | - |
| 54 | 3-COOH | 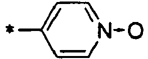 | 3 | 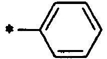 | 2 | 0 | - |
| 55 | H | 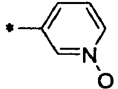 | 3 | 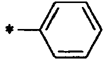 | 2 | 0 | - |
| 56 | 3-COOH | 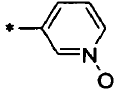 | 3 | 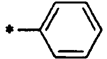 | 2 | 0 | - |

Es hat sich auch herausgestellt, dass Verbindungen der allgemeinen Formel (IV) 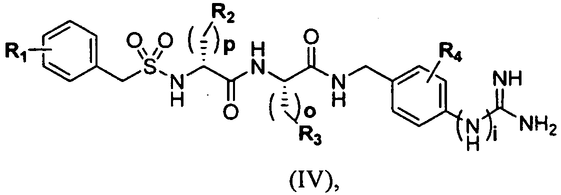 die der allgemeinen Formel (I) entspricht, mit
R₁, gegebenenfalls einfach oder mehrfach vorhanden und unabhängig voneinander ein COOR₅-Rest, mit R₅ gleich Wasserstoff oder einer verzweigten oder linearen Niederalkylgruppe mit 1-6 Kohlenstoffatomen, vorzugsweise Methyl oder Ethyl, insbesondere Methyl, ein verzweigter oder linearer Aminoalkyl-Rest mit 1-6 Kohlenstoffatomen, vorzugsweise Methyl, ein Halogen- oder Pseudohalogen-Rest, vorzugsweise Chlor oder eine Cyanogruppe, oder ein Polyethylenglykol-Rest der Formel (V) oder (VI)

CH₃-O-(CH₂-CH₂-O-)ₙ-CH₂-CH₂-(C=O)-NH-CH₂- (V)

CH₃-O-(CH₂-CH₂-O-)ₙ-CH₂-CH₂-NH-(C=O)-CH₂-CH₂-(C=O)-NH-CH₂- (VI)

mit n so definiert, dass die Polyethylenkette ein durchschnittliches Molekulargewicht von 10000 Da, 5000 Da, 3400 Da, 2000 Da, 1000 Da oder 750 Da hat, vorzugsweise ist n eine ganze Zahl von ca. 18 bis ca. 250, insbesondere ca. 18, ca. 25, ca. 50, ca. 85, ca. 125 oder ca. 250;
R₂ ein verzweigter oder linearer Alkyloxy-Rest mit 1-6 Kohlenstoffatomen, vorzugsweise tertiäres Butyl, ein Hydroxy-Rest, Amino-Rest oder ein verzweigter oder linearer Alkyloxycarbonylamido-Rest mit 1-6 Kohlenstoffatomen, vorzugsweise tertiäres Butyl, oder ein Polyethylenglykol-Rest der Formel (V) oder (VI) mit n wie oben definiert;
R₃ ausgewählt aus folgenden Resten: 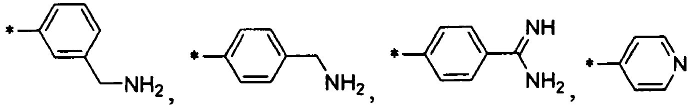 vorzugsweise 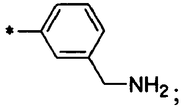 R₄ gegebenenfalls ein einfach oder mehrfach vorhandener Halogen-Rest, vorzugsweise Fluor,
o = 1 oder 2;
p = 1, 2, 3 oder 4, insbesondere 1 oder 4,
i = 0 oder 1, insbesondere 0;
sowie deren racemische Mischungen und Salze mit organischen oder anorganischen Säuren, gemäß der vorliegenden Erfindung auch geeignet sind.

Auch in diesem Fall sind Verbindungen bevorzugt, bei denen R₁ einfach und in meta- oder para-Stellung vorhanden ist, vorzugsweise ist R₁ Wasserstoff oder ein COOH-Rest, insbesondere ist R₁ einfach vorhanden und ausgewählt aus Wasserstoff, einer 4-COOH-Gruppe oder einer 3-COOH-Gruppe.

Die Salze dieser Verbindungen sind wiederum im allgemeinen gebildet aus Salzsäure, HBr, Essigsäure, Trifluoressigsäure, Toluensulfonsäure oder anderen geeigneten Säuren.

Beispiele von derartigen Verbindungen sind Verbindungen der Formel (IV) mit i = 0 und ohne Rest R₄, die folgendermaßen definiert sind:

| Verbindung Nr. | R₁ | R₂ | p | R₃ | o |
|---|---|---|---|---|---|
| 57 | H | 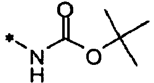 | 4 | 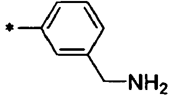 | 1 |
| 58 | 4-COOH | 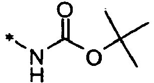 | 4 | 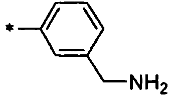 | 1 |
| 59 | H | 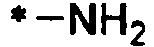 | 4 | 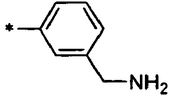 | 1 |
| 60 | H | 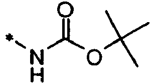 | 1 | 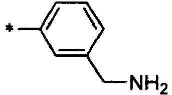 | 1 |
| 61 | H | *-NH₂ | 1 | 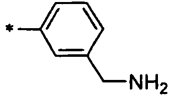 | 1 |
| 62 | H | 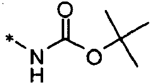 | 4 | 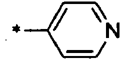 | 2 |
| 63 | H | 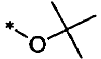 | 1 | 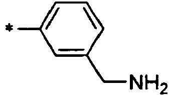 | 1 |
| 64 | 4-COOMe | 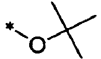 | 1 | 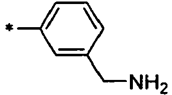 | 1 |
| 65 | 4-COOH | 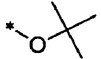 | 1 | 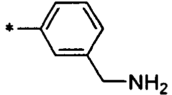 | 1 |
| 66 | 3-COOMe | 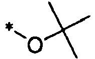 | 1 | 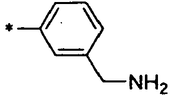 | 1 |
| 67 | 3-COOH | 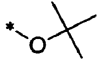 | 1 | 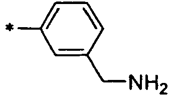 | 1 |
| 68 | H | *-OH | 1 | 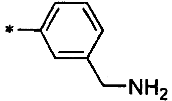 | 1 |
| 69 | 4-COOMe | *-OH | 1 | 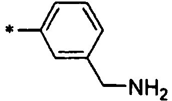 | 1 |
| 70 | 4-COOH | *-OH | 1 | 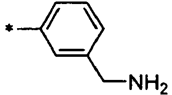 | 1 |
| 71 | H | 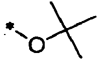 | 1 | 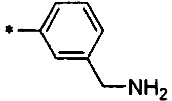 | 1 |
| 72 | H | *-OH | 1 | 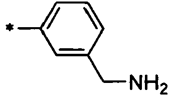 | 1 |
| 73 | H | 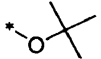 | 1 | 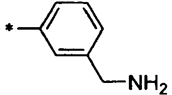 | 2 |
| 74 | H | *-OH | 1 | 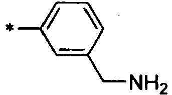 | 2 |
| 75 | H | 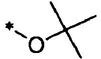 | 1 | 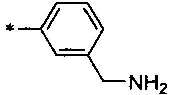 | 1 |
| 76 | H | *-OH | 1 | 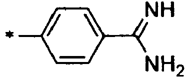 | 1 |
| 77 | H | 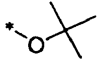 | 1 | 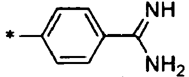 | 2 |
| 78 | H | *-OH | 1 | 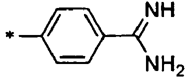 | 2 |

Die Verbindungen der allgemeinen Formel 1 können in prinzipiell bekannter Weise, wie nachfolgend beschrieben, beispielsweise wie folgt hergestellt werden, wobei im allgemeinen sequentiell die entsprechenden Aminosäuren an ein an der Amidinogruppe geschütztes Amidinobenzylamin angekoppelt werden. Hierbei trägt die N-terminale Aminosäure entweder den P4-Rest bereits oder dieser wird anschließend daran gebunden.

Die Nomenklatur der einzelnen Bestandteile P1, P2, P3 und P4 der erfindungsgemäßen Verbindungen ist nachfolgend ersichtlich (siehe auch Schechter und Berger, 1967). Beispielsweise wird aus dem kommerziell erhältlichen 4-Cyanobenzylamin (Showa Denko K.K., Japan) das geschützte, vorzugsweise Boc-geschützte, und an der Amidinogruppe geschützte Amidinobenzylamin, insbesondere 4-Acetyloxamidino-benzylamin nach dem Fachmann bekannten Verfahren gewonnen. Nach Abspaltung der Schutzgruppe erfolgt die Ankopplung der weiteren Aminosäuren und des P4-Restes mittels Standardkopplungsmethoden und Schutzgruppen, vorzugsweise mit Boc als N-terminaler Schutzgruppe. Die P3-Aminosäure kann auch direkt als geschützte, vorzugsweise Benzylsulfonyl-geschützte, Aminosäure, die den R1-Rest schon trägt, gekoppelt werden. Die Peptidanaloga werden sequentiell, beginnend vom Acetyloxamidinobenzylamin, aufgebaut. Die meisten Zwischenprodukte kristallisieren gut und lassen sich damit einfach reinigen. Die Endreinigung der Hemmstoffe erfolgt auf der letzten Stufe vorzugsweise über präparative, reversed-phase HPLC.

Ein weiterer Gegenstand der Erfindung ist daher ein Verfahren zur Herstellung einer erfindungsgemäßen Verbindung, wobei sequentiell die entsprechenden Aminosäuren an ein an der Amidino- oder Guanidinogruppe geschütztes Amidino- oder Guanidinobenzylamin, beispielsweise an ein 4-Acetyloxamidinobenzylamin oder an ein 4-(Benzyloxycarbonylamidino)benzylamin, angekoppelt werden, wobei die N-terminale Aminosäure entweder den P4-Rest bereits trägt oder dieser anschließend daran gebunden wird. Nach einer eventuellen Reinigung können die erhaltenen Verbindungen gegebenenfalls PEGyliert werden.

Ein beispielhaftes Verfahren zur Herstellung der erfindungsgemäßen Verbindungen umfasst folgende Schritte:
(a) Amidierung einer entsprechenden Nα-geschützten Aminosäure mit dem Rest R₃ mit einem entsprechenden geschütztem Aminomethylbenzamidin oder -guanidin,
(b) nach Abspaltung der Nα-Schutzgruppe der Aminosäure mit R3 Umsetzung des erhaltenen Produktes mit der entsprechenden Benzylsulfonylaminosäure mit den Resten R₁ und R₂ und Abspaltung restlicher Schutzgruppen zur erfindungsgemäßen Verbindung, und nach einer möglichen Reinigung
(c) gegebenenfalls die erhaltene Verbindung PEGyliert wird.

Weitere dem Fachmann allgemein bekannte Verfahrenseinzelheiten, z. B. zu den gewählten Schutzgruppen oder der PEGylierung, können den Beispielen entnommen werden. Eine bevorzugte Schutzgruppe des Amidstickstoffes ist beispielsweise tert.-Butyloxycarbonyl (Boc). Die Ausgangsverbindungen sind z. B. Aminosäure-Derivate bzw. PEG-Derivate. Die Chemikalien sind im allgemeinen käuflich erhältlich. Die PEGylierung, d. h. die Derivatisierung mit Polyethylenglykol, erfolgte im Allgemeinen entweder über die P3-Aminosäure oder über den P4-Benzylsulfonylrest mit aktivierten PEG-Derivaten, z. B. mit als N-Hydroxysuccinimidester-aktiviertem PEG.

Eine vorteilhafte Eigenschaft der PEG-gekoppelten Verbindungen ist die Verlängerung der Halbwertszeit der Inhibitoren in der Blutzirkulation. Die folgende Struktur zeigt ein Beispiel, bei dem die PEG-Kette über die P3-Aminosäure (D-Lys) gekoppelt wurde.

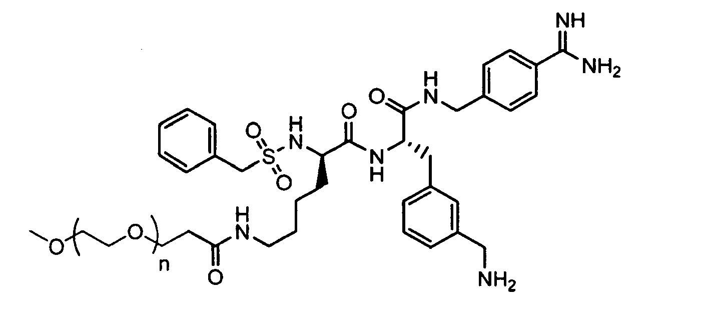

Unter Verwendung eines Succinyl-Linkers wurde die folgende Verbindung erhalten:

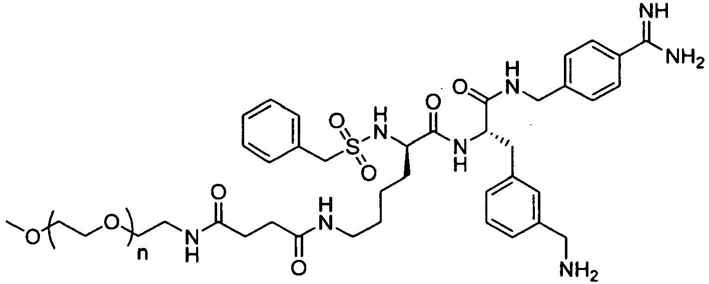

Zusätzlich wurde die PEG-Kette über einen geeigneten P4-Benzylsulfonylrest entsprechend der unten dargestellten allgemeinen Formel angekoppelt, wobei der P4-Rest mit einer Aminomethylgruppe in para- oder ortho-Stellung modifiziert war.

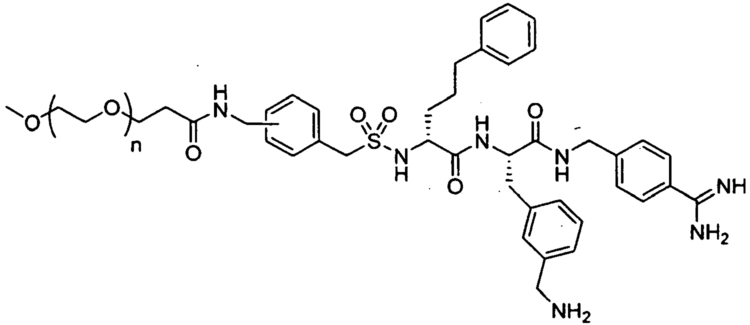

Unter Verwendung eines Succinyl-Linkers wurde die folgende Verbindung erhalten:

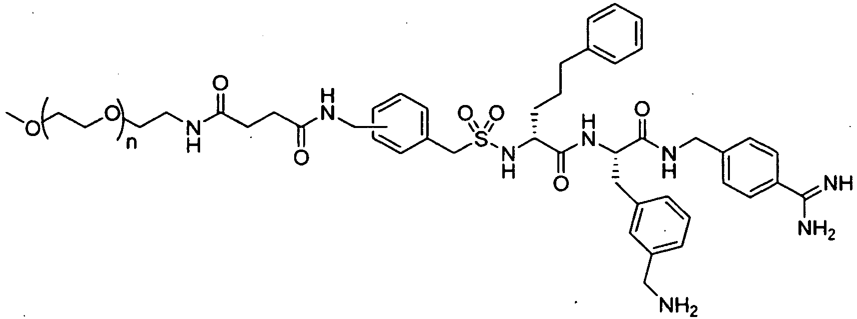

Dem Fachmann sind jedoch auch andere Herstellungsverfahren bekannt, die gleichermaßen durchgeführt werden können. Die PEGylierten Verbindungen sind im allgemeinen Mischungen von Verbindungen verschiedener PEGylierungsgrade, wobei das Molekulargewicht der PEG-Reste üblicherweise im Bereich von 750, 1000, 2000, 3400, 5000 oder 10000 Da liegt. Es sind jedoch auch diskrete Polyethylenglykole mit definiertem Molekulargewicht käuflich erhältlich.

Die vorliegende Erfindung erstreckt sich auch auf ein Arzneimittel enthaltend mindestens eine der erfindungsgemäßen Verbindungen, vorzugsweise zur Behandlung von Blutverlust, insbesondere bei hyperfibrinolytischen Zuständen, bei Organtransplantationen oder herzchirurgischen Eingriffen vor allem mit cardiopulmonalem Bypass.

Die vorliegende Erfindung umfasst auch einen Fibrinkleber, der mindestens eine der erfindungsgemäßen Verbindungen enthält, bei dem Aprotinin durch einen geeigneten Hemmstoff der vorliegenden Erfindung ersetzt wird.

Unter Fibrinkleber wird im allgemeinen ein physiologischer Zweikomponentenklebstoff verstanden, der als erste Komponente Fibrinogen, Faktor XIII und Aprotinin bzw. mindestens eine der erfindungsgemäßen Verbindungen und als zweite Komponente Thrombin und Calciumchlorid zur Faktor XIII Aktivierung enthält.

Die vorliegende Erfindung betrifft auch die Verwendung mindestens einer erfindungsgemäßen Verbindung zur Herstellung eines erfindungsgemäßen Arzneimittels oder eines erfindungsgemäßen Fibrinklebers nach dem Fachmann allgemein bekannten Verfahren, z. B. durch Mischen mit geeigneten Hilfs- oder Zusatzstoffen.

Die folgenden Beispiele sollen die Erfindung näher erläutern ohne sie zu beschränken.

### Beispiele

### 1. Analyseverfahren

### 1.1 Analytische HPLC

Zur analytischen reversed-phase-HPLC wurde eine HPLC-Anlage LC-10A der Firma Shimadzu, bestehend aus den Teilsystemen CTO-10AS Säulenofen, LC-10AD Pumpen (2 x), DGU-14A Degaser, SIL-10AD Autoinjektor, SCL-10A Systemcontroller, SPD-10A UV-Vis Detektor und einer Säule Luna 5 µm C18(2) 100 Å, 250 x 4,6 mm der Firma Phenomenex, unter Benutzung der zugehörigen Software Shimadzu CLASS-VP, Version 5.3, verwendet. Die Detektion erfolgte bei 220 nm. Als Elutionsmittel dienten Wasser mit 0,1 % TFA (A) und Acetonitril mit 0,1 % TFA (B) bei einer Flussrate von 1 ml/min und einem linearen Gradienten (1 % B/min). Je nach Verbindung, wurden unterschiedliche Startbedingungen für die analytische HPLC verwendet, die bei den entsprechenden Verbindungen angegeben sind.

Zur Analyse sämtlicher Polyethylenglykol-modifizierten Wirkstoffe wurde eine Jupiter-Säule 5 µm C18(2) 300 Å, 250 x 4,6 mm der Firma Phenomenex verwendet.

### 1.2 Präparative HPLC

Zur präparativen RP-HPLC wurde eine HPLC-Anlage der Firma Shimadzu, bestehend aus den Teilsystemen LC-8A präparative Pumpen (2 x), DGU-14A Degaser, FRC-10A Fraktionssammler, SCL-10A Systemcontroller, SPD-10A UV-Vis Detektor und einer Säule Luna 5 µm C8(2) 100 Å, 250 x 30,0 mm der Firma Phenomenex, unter Benutzung der zugehörigen Software Shimadzu CLASS-VP, Version 5.3, verwendet. Die Detektion erfolgte bei 220 nm. Als Elutionsmittel dienten ebenfalls Wasser mit 0,1 % TFA (A) und Acetonitril mit 0,1 % TFA (B) bei einer Flussrate von 10 oder 20 ml/min und einem geeigneten Gradienten.

### 1.3 Massenspektroskopie

Die Massenspektren wurden standardmäßig auf einem ESI-MS LCQ der Firma Finnigan (Bremen, Deutschland), gemessen. Sämtliche Polyethylenglykol-gekoppelten Verbindungen wurden auf einem Maldi Ultraflex Tof/Tof Gerät der Firma Bruker analysiert.

### Verwendete Abkürzungen

- ACN: Acetonitril
- 4-Amba: 4-Amidinobenzylamid
- Ame: Aminomethyl
- Boc: tert.-Butyloxycarbonyl
- BSA: bovines Serumalbumin
- Bzl: Benzyl
- Bzls: Benzylsulfonyl
- DIEA: Diisopropylethylamin
- DCM: Dichlormethan
- DMF: N,N-Dimethylformamid
- HBTU: 2-(1H-Benzotriazol-1-yl)-1,1,3,3-Tetramethyluroniumhexa-fluorophosphat
- HPLC: Hochleistungs-Flüssigkeitschromatographie
- i.V.: im Vakuum
- LM: Lösungsmittel
- MS: Massenspektroskopie
- ONHS: N-Hydroxysuccinimidester
- NMM: N-Methylmorpholin
- PEG: Polyethylenglykol
- Phe(3-Ame): 3-Aminomethylphenylalanin
- Ppg: Phenylpropylglycin
- RT: Raumtemperatur
- tBu: tert.-Butyl
- Tfa: Trifluoracetyl
- TFA: Trifluoressigsäure
- TEA: Triethylamin
- TMS-Cl: Trimethylsilylchlorid
- Me: Methyl

### 2. Synthese der Inhibitoren

### 2.1 3-HOOC-Bzls-d-Ppg-Phe(3-Ame)-4-Amba x 2 Acetat (3)

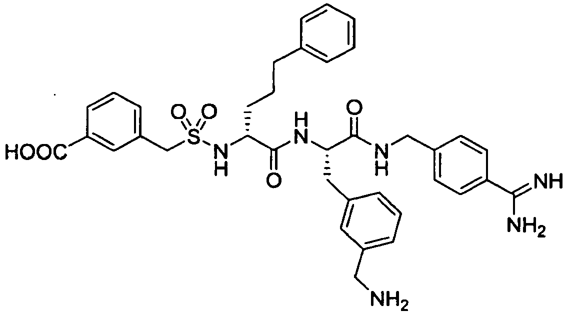

a) Boc-Phe(3-Ame)-OH x Acetat 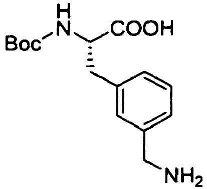 5 g (17,2 mmol) Boc-Phe(3-CN)-OH (Acros Organics) wurden in 700 ml 90 %iger Essigsäure gelöst und 3 Stunden unter Normaldruck mit Wasserstoff und 800 mg 10 % Pd/C als Katalysator bei 40°C hydriert. Das LM wurde i.V. entfernt, der Rückstand in einer kleinen Menge Methanol gelöst und durch Zugabe von Diethylether gefällt. Ausbeute: 4,1 g (HPLC: 16,7 min, Start bei 10 % B)
b) Boc-Phe(3-Tfa-Ame)-OH 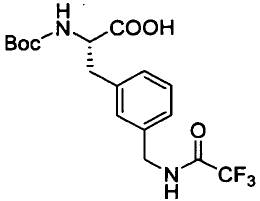 4,6 g (13 mmol) Boc-Phe(3-Ame)-OH x Acetat wurden in 30 ml Methanol gelöst und mit 4 ml (29,9 mmol) DIEA und 2 ml (16,78 mmol) Trifluoressigsäureethylester bei Raumtemperatur versetzt. Der Ansatz wird gerührt, nach ca. 15 min ist die ursprüngliche Suspension komplett gelöst. Nach einer Stunde wird das LM i.V. entfernt und der Rückstand in Essigester und Wasser gelöst. Die Essigesterphase wird 2x mit 5% KHSO₄-Lösung und 3 x mit gesättigter NaCl-Lösung gewaschen und die organische Phase mit Na₂SO₄ getrocknet. Das LM wird i.V. entfernt.
   Ausbeute: 4,9 g amorpher Feststoff (HPLC: 28,13 min, Start bei 20 % B)
c) Boc-Phe(3-Tfa-Ame)-4-(Acetylhydroxyamidino)-Benzylamid 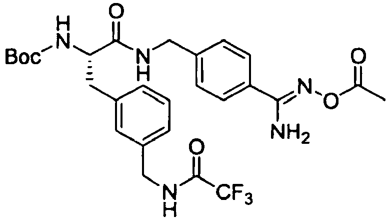 5,43 g (13,9 mmol) an Boc-Phe(3-Tfa-Ame)-OH und 4,28 g (15,3 mmol) 4-(Acetylhydroxy-amidino)-Benzylamin (Synthese beschrieben im Supplement zu Schweinitz et al., 2004) wurden in 50 ml DMF gelöst und bei 0 °C mit 5,2 ml (30 mmol) DIEA und 5,81 g (15,3 mmol) HBTU versetzt. Der Ansatz wird 15 min bei 0 °C und weitere 3 h bei RT gerührt. Das LM wird i.V. entfernt und der Rückstand in Essigester gelöst. Die Essigesterphase wird 3 x mit 5 % KHSO₄-Lösung, 1 x mit gesättigter NaCl-Lösung, 3 x mit gesättigter NaHCO₃-Lösung und 2 x mit gesättigter NaCl-Lösung gewaschen. Das zwischen den Phasen ausgefallene Produkt wird abgesaugt und i.V. getrocknet.
   Ausbeute: 4,17 g weiße Kristalle (HPLC: 28,08 min, Start bei 20 % B)
d) H-Phe(3-Tfa-Ame)-4-(Acetylhydroxyamidino)-Benzylamid x HCl 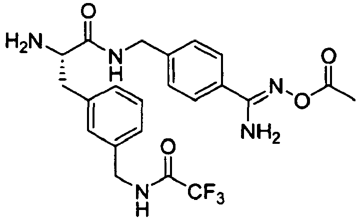 4,1 g Boc-Phe(3-Tfa-Ame)-4-(Acetylhydroxyamidino)-Benzylamid wurden in 60 ml trockenem Dioxan suspendiert und mit 11 ml 4 N HCl in Dioxan versetzt. Nach kurzer Ultraschallbehandlung wird der Ansatz 1 h bei Raumtemperatur geschüttelt. Nach 1 h wird das Produkt durch Zugabe von Diethylether gefällt, abgesaugt und i.V. getrocknet.
   Ausbeute: 3,8 g weißer Feststoff (HPLC: 9,47 min, Start bei 20 % B)
e) 3-MeOOC-Bzl-SO₃⁻ x Na⁺ 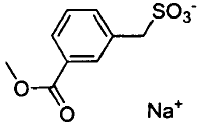 5 g (21.8 mmol) 3-Bromomethylbenzoesäure-Methylester (Acros Organics) wurden in 25 ml Wasser suspendiert und mit 2.94 g (23.8 mmol) Na₂SO₃ vesetzt. Der Ansatz wurde 5 h unter Rückfluß erhitzt und danach das LM teilweise i.V. entfernt, bis Kristallisation einsetzte. Der Ansatz wurde über Nacht bei 4 °C aufbewahrt und das Produkt abfiltriert.
   Ausbeute: 3.7 g weiße Kristalle (HPLC: 12,02 min, Start bei 10 % B)
f) 3-MeOOC-Bzls-Cl 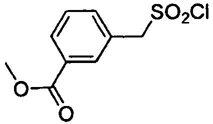 2,5 g (9,91 mmol) an 3-MeOOC-Bzl-SO₃⁻ x Na⁺ wurden mit Phosphorylchlorid angefeuchtet und mit 2,27 g (10,9 mmol) PCl₅ versetzt. Der Ansatz wurde ca. 5 min bei 0 °C gekühlt und danach 4 h auf dem Ölbad erhitzt (Badtemperatur 80 °C). Danach wurde der Ansatz auf Eis gegossen und kräftig gerührt. Nach ca. 30 min Rühren beginnt das Säurechlorid auszufallen, wird abgesaugt und i.V. getrocknet.
   Ausbeute: 1,4 g weißer Feststoff
g) 3-MeOOC-Bzls-d-Ppg-OH 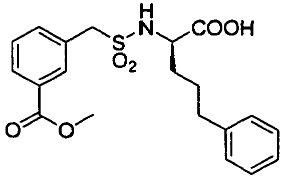 1,3 g (6,72 mmol) H-d-Ppg-OH (Peptech, Burlingtom, MA) wurden in 90 ml trockenem DCM suspendiert und mit 2 ml (15,7mol) TMS-Cl und 2,6 ml (15mmol) DIEA versetzt. Der Ansatz wurde 1 h unter Rückfluss erhitzt, die klare Lösung auf 0 °C abgekühlt und mit 2 g (8 mmol) an 3-MeOOC-Bzls-Cl und 2,6 ml DIEA versetzt. Der Ansatz wurde 15 min bei 0 °C und 1,5 h bei RT gerührt. Das LM wurde i.V. entfernt und der Rückstand in 700 ml halbgesättigter NaHCO₃-Lösung gelöst. Der Ansatz wurde 2x mit wenig Essigester extrahiert und danach die wässrige Phase mit HCl angesäuert (pH ca. 2-3). Der Ansatz wird 3 x mit 150 ml Essigester extrahiert und die vereinte Essigesterphase 2 x mit 5 % KHSO₄-Lösung und 1 x mit gesättigter NaCl-Lösung gewaschen. Die organische Phase wird mit Na₂SO₄ getrocknet und das LM i.V. entfernt.
   Ausbeute: 2,4 g Öl (HPLC: 33,53 min, Start bei 20 % B)
h) 3-MeOOC-Bzls-d-Ppg-Phe(3-Tfa-Ame)-4-(Acetylhydroxyamidino)-Benzylamid 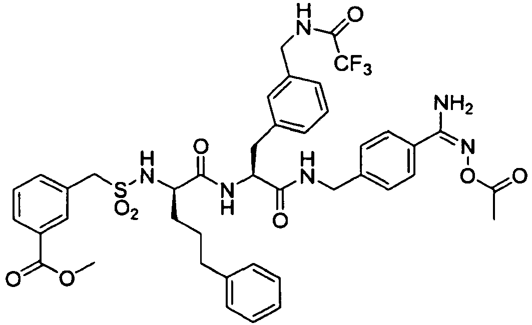 0,605g (1,5 mmol) 3-MeOOC-Bzls-d-Ppg-OH und 0,85g (1,65mmol) H-Phe(3-Tfa-Ame)-4-(Acetylhydroxyamidino)-Benzylamid x HCl wurden in 40 ml trockenem DMF gelöst und bei 0 °C mit 0,63g (1,65 mmol) HBTU und 0,6 ml (0,34 mmol) DIEA vesetzt. Der Ansatz wird 15 min bei 0 °C und weitere 3 h bei RT gerührt. Das LM wird i.V. entfernt und der Rückstand in Essigester gelöst. Die Essigesterphase wird 3 x mit 5 % KHSO₄-Lösung, 1 x mit gesättigter NaCl-Lösung, 3 x mit gesättigter NaHCO₃-Lösung und 2 x mit gesättigter NaCl-Lösung gewaschen. Das LM wird i.V. entfernt.
   Ausbeute: 1,36 g Öl (HPLC: 38,40 min, Start bei 20 % B)
i) 3-MeOOC-Bzls-d-Ppg-Phe(3-Tfa-Ame)-4-Amidinobenzylamid x Acetat 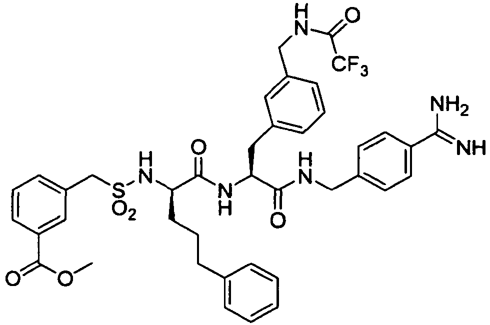 1,3 g 3-MeOOC-Bzls-d-Ppg-Phe(3-Tfa-Ame)-4-(Acetylhydroxyamidino)-Benzylamid werden in 100 ml 90 % Essigsäure gelöst und über Nacht bei Normaldruck mit Wasserstoff und 150 mg 10 % Pd/C als Katalysator hydriert. Der Katalysator wird abfiltriert und das Filtrat i.V. eingeengt.
   Ausbeute: 1,2 g Öl (HPLC: 29,45 min, Start bei 20 % B)

### 2.2 3-HOOC-Bzls-d-Ppg-Phe(3-Ame)-4-Amidinobenzylamid x 2 Acetat (3)

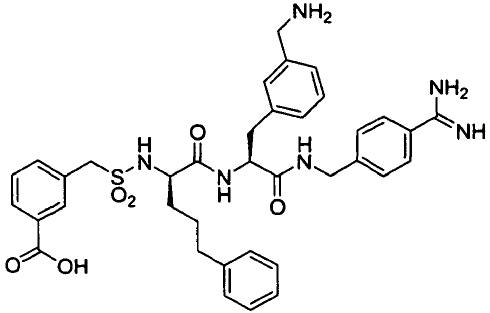

1,2 g 3-MeOOC-Bzls-d-Ppg-Phe(3-Tfa-Ame)-4-Amidinobenzylamid x Acetat wurden in einer Mischung aus 10 ml Dioxan und 10 ml 1 N LiOH 1,5 h gerührt. Anschließend wurde der Ansatz durch Zugabe von TFA neutralisiert und das Produkt mittels präparativer *reversed-phase* HPLC gereinigt. Die Produkt-enthaltenden Fraktionen wurden vereinigt und lyophilisiert.

Ausbeute: 0,4 g als TFA-Salz (HPLC: 24,16 min, Start bei 10 % B)

MS: berechnet: 698,29 gef.: 699,3 (M+H)⁺

Das Produkt wurde mittels präparativer HPLC durch Elution mittels eines ansteigenden Acetonitril-Gradienten, enthaltend 0,1 % Essigsäure, in das Acetat-Salz überführt.

Ausbeute: 0,32 g

Entsprechend der obigen Synthesebeschreibung wurden weitere Inhibitoren synthetisiert, wobei unterschiedlich substituierte oder unsubstituierte Benzylsulfonyl-Reste und verschiedene P3-Aminosäuren als Ersatz für d-Phenylpropylglycin eingebaut wurden. Weitere Analoga des d-Phenylpropylglycins wurden mittels Heck-Kopplung synthetisiert und in P3-Position der Inhibitoren eingebaut. Die Synthese kann beispielsweise wie folgt durchgeführt werden:

### 2.3 Bzls-d-Gly(3-Cl-Phpr)-Phe(3-Ame)-4-Amba x 2 TFA (6)

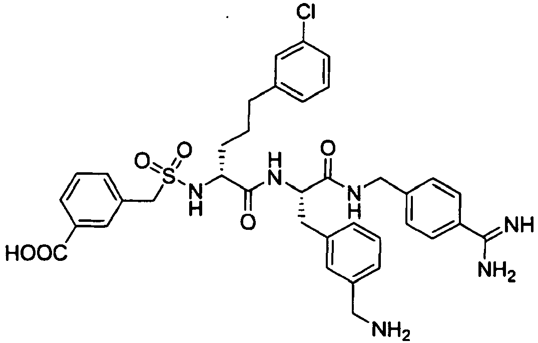

a) Bzls-d-Gly(Allyl)-OH 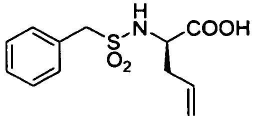 1,0 g (8,68 mmol) D-Allylglycin (Peptech, Burlingtom, MA) wurden in 50 ml trockenem DCM suspendiert und mit 2,4 ml (19 mmol) TMS-Cl und 3,3 ml (19 mmol) DIEA versetzt. Der Ansatz wurde 1 h unter Rückfluss erhitzt, die klare Lösung auf 0 °C abgekühlt und mit 2,35 g (9,55 mmol) Bzls-Cl und 1,8 ml DIEA versetzt. Der Ansatz wurde 15 min bei 0 °C und 1,5 h bei RT gerührt. Das LM wurde i.V. entfernt und der Rückstand in 700 ml halbgesättigter NaHCO₃-Lösung gelöst. Der Ansatz wurde 2 x mit Essigester extrahiert und danach die wässrige Phase mit HCl angesäuert (pH ca. 2-3). Der Ansatz wurde 3 x mit 150 ml Essigester extrahiert und die vereinte Essigesterphase 2 x mit 5 % KHSO₄-Lösung und 1 x mit gesättigter NaCl-Lösung gewaschen. Die organische Phase wurde mit Na₂SO₄ getrocknet und das LM i.V. entfernt.
   Ausbeute: 2,2 g Öl (HPLC: 21,1 min, Start bei 20 % B)
   MS (ESI, m/e): 267 [M-1]⁻
b) Bzls-d-Ala(3-Cl-Styryl)-OH

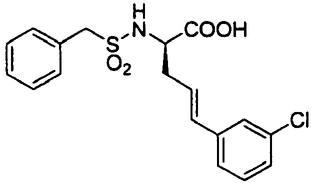

Eine Suspension von 0,476 g (1,48 mmol) Tetra-n-butylammoniumbromid, 0,34 g (4,05 mmol) NaHCO₃, 0,32g (1,34 mmol) 1-Cl-3-Iodbenzen und 9 mg (0,04 mmol) Palladium(II)acetat in einer Mischung aus 2,5 ml DMF und 2,5 ml Wasser wird 10 min bei RT gerührt. Zu dieser Suspension wird eine Lösung von 0,4 g (1,48 mmol) Bzls-d-Gly(Allyl)-OH in 2,5 ml DMF und 2,5 ml Wasser zugegeben und der Ansatz 4-6 Tage auf 45-50°C erwärmt, ggf. werden wiederholt geringe Mengen Katalysator nachdosiert. Der Katalysator wird abfiltriert, das LM i.V. entfernt und der Rückstand in 50 ml 5 % KHSO₄-Lösung suspendiert. Der Ansatz wird 3 x mit je 15-20 ml Essigester extrahiert und die vereinte Essigesterphase 2 x mit 5 % KHSO₄-Lösung und 1 x mit gesättigter NaCl-Lösung gewaschen. Die organische Phase wird mit Na₂SO₄ getrocknet und das LM i.V. entfernt. Der Rückstand (0,55 g dunkles Öl) wird mittels Flashchromatographie an Kieselgel 60 (40-63 µM) gereinigt (Gradient 0-20 % Methanol in DCM).

Ausbeute: 0,23 g (HPLC: 39,7 min, Start bei 20 % B)

Der weitere Aufbau des Inhibitors erfolgte analog der beschriebenen Synthese für Hemmstoff 1. Das Intermediat 2.2.b wurde mit dem Zwischenprodukt 2d (H-Phe(3-Tfa-Ame)-4-(Acetylhydroxyamidino)-Benzylamid x HCl) analog Vorschrift 2h gekoppelt. Die Hydrierung des erhaltenen Intermediates erfolgte analog Vorschrift 2i, dabei wurde keinerlei Abspaltung des Cl-Atoms der P3-Aminosäure beobachtet. Im letzten Schritt erfolgte die Abspaltung der Trifluoracetyl-Schutzgruppe mittel LiOH in Dioxan analog dem finalen Syntheseschritt bei der Herstellung von Verbindung 3.

HPLC: 29,04 min, Start bei 10 % B

MS: berechnet: 688,26 gef.: 689,2 (M+H)⁺

### 2.4 3-HOOC-Bzls-d-Ppg-Phe(3-Ame)-4-Guanidinobenzylamid x 2 Acetat (4)

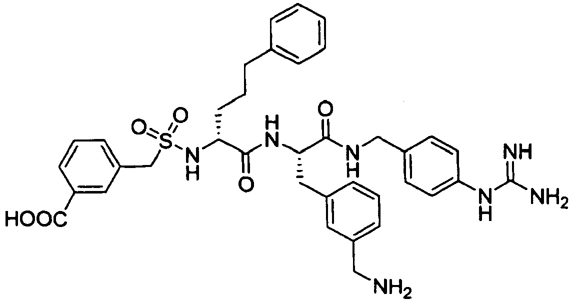

Analog der obigen Synthesebeschreibung 2.2a-i wurde auch Verbindung 4 synthetisiert, wobei anstelle des 4-(Acetylhydroxyamidino)-Benzylamid p-Nitrobenzylamin für Schritt c) verwendet wurde. Die Reduktion des Nitrobenzylamin-Restes zum p-Aminobenzylamin. erfolgte analog Schritt 2.2i mit Methanol/THF (1:1) als Lösungsmittel. Die Guanylierung des p-Aminobenzylamin-Restes erfolgte mit kommerziell erhältlichem 1,3-di-Boc-2-(trifluoromethylsulfonyl)guanidin (Fluka) als Guanylierungsreagenz. Dazu wurde das Intermediat aus der Reduktion in Dioxan gelöst und mit dem Guanylierungsreagenz und TEA bei 50 °C einen Tag gerührt. Nach Abziehen des Lösungsmittels wurden die Boc-Schutzgruppen in bekannter Weise mit TFA abgespalten. Nach Abziehen des Lösungsmittels erfolgte im letzten Schritt die Abspaltung der Trifluoracetyl-Schutzgruppe und des Methylesters mittels LiOH in Dioxan analog dem finalen Syntheseschritt bei der Herstellung von Verbindung 3.

HPLC: 25,1 min, Start bei 10 % B

MS: berechnet: 713,3 gef.: 714,4 (M+H)⁺

### 2.5 Pegylierte Verbindungen

Mittels Standardverfahren wurden weitere Hemmstoffe synthetisiert, an die Polyethylenglykol(PEG)ketten unterschiedlicher Kettenlänge kovalent gekoppelt wurden. Für sämtliche Synthesen wurden kommerziell erhältliche PEG-Derivate der Firmen Fluka, Nektar Therapeutics oder Rapp Polymere mit unterschiedlichen durchschnittlichen Molekulargewichten (1000 Da, 2000 Da, 5000 Da, 10000 Da) verwendet. Die verwendeten PEG-Derivate sind an einem Ende als Methylether geschützt und am anderen Ende mit einem als N-Hydroxysuccinimidester-aktiviertem Propionsäure- oder Bernsteinsäurerest modifiziert. Dadurch konnten diese aktivierten PEG-Derivate mit einer freien Aminogruppe des Inhibitors umgesetzt werden (siehe Syntheseschemata 1 bis 5). Im letzten Schritt wurde durch Versetzen mit 1 N NaOH-Lösung die TFA-Schutzgruppe abgespalten und die Produkte über Ionenaustauschchromatographie an Fractogel^{®} CE (Merk KGaA, Darmstadt) mittels eines Ammoniumacetat-Gradienten gereinigt und 3 x aus Wasser lyophilisiert. Die nachfolgenden Beispiele ergaben Inhibitoren mit einer PEG-Kette einer durchschnittlichen Masse von ca. 1000, 2000, 5000 oder 10000 Da.

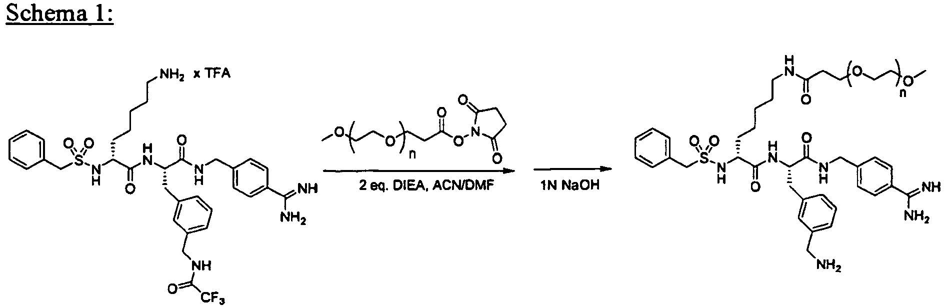

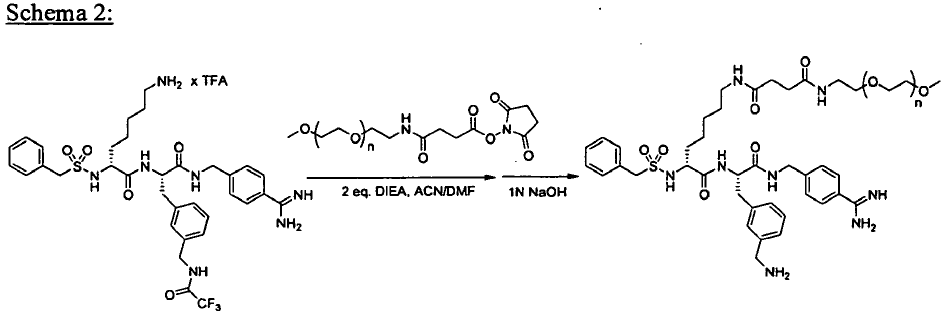

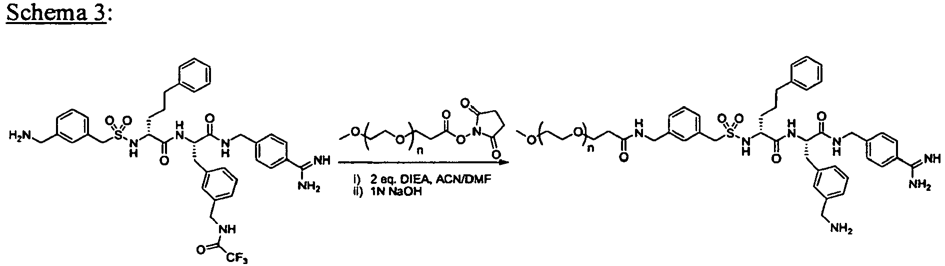

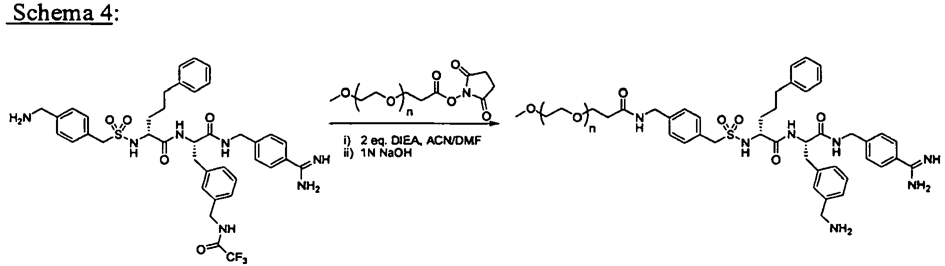

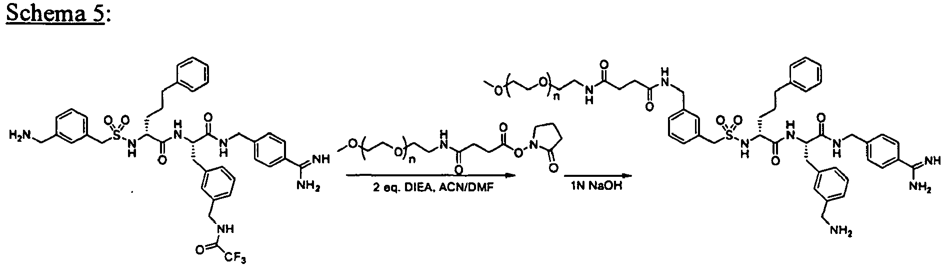

Die gemäß den Beispielen synthetisierten Verbindungen einschließlich ihrer Hemmkonstanten sind in der unteren Tabelle zusammengefasst.

### 3. Bestimmung der Hemmkonstanten für Plasmin und PK (Kᵢ-Werte in nM)

Die Bestimmung der Hemmwirkung für die einzelnen Enzyme erfolgte analog einer bereits bekannten Methode (Stürzebecher et al., 1997).

Die Reaktionen zur Bestimmung der Hemmung von human-Plasmin und human-Plasmakallikrein wurden in folgendem Ansatz bei 25 °C durchgeführt:
- 200 µl TBS (0,05 M Trishydroxymethylaminomethan; 0,154 M NaCl, 2 % Ethanol, pH 8,0; enthält den Inhibitor)
- 25 µl Substrat (2 mM, 1 mM und 0,67 mM Tosyl-Gly-Pro-Lys-pNA = Chromozym PL Firma LOXO für Plasmin, und 2 mM, 1 mM und 0,5 mM H-D-Pro-Phe-Arg-pNA = S2302 Firma Chromogenix für PK, gelöst in H₂O)
- 50 µl Enzymlösung (Plasmin Fa. Calbiochem: 2-5 mU/ml in 0,154 M NaCl + 0, 1 % BSA m/v + 25% v/v Glycerol; Plasmakallikrein Firma Enzyme Research Lab.: 20-60 ng/ml in 0,154 M NaCl + 0,1 % BSA m/v)

Für Kinetik nullter Ordnung wurde nach 20 min die Reaktion durch Zugabe von 25 µl Essigsäure (50% v/v) gestoppt und die Absorption bei 405 nm mittels eines Microplate Reader (Multiscan Ascent, Firma Thermo) bestimmt. Im Falle von Kinetik pseudoerster Ordnung wurden die Reaktionsgeschwindigkeiten im Gleichgewichtszustand durch Aufnahme der Reaktionskinetiken erfasst. Die Kᵢ-Werte wurden entweder nach Dixon (1953) durch lineare Regression mittels eines Computerprogramms oder durch Parameteranpassung entsprechend der Geschwindigkeitsgleichung für kompetitive Inhibierung ermittelt. Die Kᵢ-Werte sind das Mittel aus mindestens zwei Bestimmungen.

### Tabelle

Kᵢ-Werte: A bedeutet <10 nM, B bedeutet <100 nM, C bedeutet <1000 nM und D bedeutet >1000 nM

**1. Gruppe**

| Nr. | Masse (gefunden/berechnet) | HPLC %AN | Plasmin [K*ᵢ*] | PK [K*ᵢ*] | Xa [K*ᵢ*] | Thrombin [K*ᵢ*] |
|---|---|---|---|---|---|---|
| 1 | 655,3/654,3 | 37,4 | A | A | B | C |
| 2 | 699,5/698,3 | 33,6 | A | A | B | C |
| 3 | 699,3/698,3 | 34,1 | A | A | B | D |
| 4 | 714,4/713,3 | 35,1 | B | A | D | D |
| 5 | 717,3/716,3 | 34,9 | A | A | B | D |
| 6 | 689,2/688,3 | 39,8 | A | A | B | C |
| 7 | 689,3/688,26 | 41,9 | A | A | A | B |
| 8 | 689,3/688,26 | 39,0 | A | A | B | B |
| 9 | 669,3/668,3 | 39,3 | A | A | B | C |
| 10 | 685,6/684,3 | 37,2 | A | A | B | C |
| 11 | 723,2/722,3 | 41,6 | A | A | A | C |
| 12 | 739,4/738,3 | 44,4 | A | A | B | C |
| 13 | 683,3/682,3 | 41,9 | B | A | B | C |
| 14 | 703,3/702,3 | 40,7 | A | A | B | C |
| 15 | 677,3/676,3 | 37,4 | A | A | B | B |
| 16 | 721,2/720,3 | 34,3 | A | A | C | C |
| 17 | 677,3/676,3 | 37,7 | A | A | B | C |
| 18 | 721,1/720,3 | 35,6 | A | A | B | D |
| 19 | 721,2/720,3 | 34,6 | A | A | C | C |
| 20 | 633,8/632,3 | 36,1 | A | A | B | C |
| 21 | 661,4/660,3 | 41,5 | A | A | B | C |
| 22 | 703,4/702,3 | 38,4 | A | A | B | A |
| 23 | 747,3/746,3 | 36,3 | A | A | B | B |
| 24 | 657,3/656,3 | 29,1 | A | A | A | C |
| 25 | 747,3/746,3 | 36,3 | A | A | B | D |
| 26 | 671,4/670,3 | 30,7 | A | A | A | C |
| 27 | 642,3/641,3 | 42,4 | A | A | A | C |
| 28 | 655,4/654,3 | 37,0 | A | A | C | C |
| 29 | 699,4/698,3 | 31,8 | A | A | C | C |
| 30 | 699,4/698,3 | 34,2 | A | A | C | D |
| 31 | 669,4/668,3 | 36,9 | A | B | C | B |
| 32 | 616,2/615,3 | 35,3 | A | B | C | B |
| 33 | 660,3/659,3 | 31,9 | A | B | C | D |
| 34 | 640,4/639,3 | 52,7 | A | A | C | B |
| 35 | 684,3/683,3 | 48,6 | A | A | C | C |
| 36 | 684,3/683,3 | 49,8 | A | A | B | C |
| 37 | 647,9/646,3 | 39,3 | B | B | B | B |
| 38 | 660,6/659,4 | 39,3 | A | B | B | A |
| 39 | 641,5/640,3 | 36,1 | A | A | B | A |
| 40 | 685,5/684,3 | 32,2 | A | A | C | B |
| 41 | 657,5/656,3 | 40,0 | A | A | C | B |
| 42 | 701,5/700,3 | 35,4 | B | A | D | C |
| 43 | 619,2/618,3 | 35,1 | A | B | B | A |
| 44 | 627,2/626,3 | 33,7 | B | B | B | B |
| 45 | 638,8/637,3 | 30,6 | A | A | C | B |
| 46 | 638,7/638,2 | 34,0 | A | A | C | B |
| 47 | 643,3/642,3 | 28,0 | A | A | A | A |
| 49 | 641,4/640,3 | 33,7 | A | A | B | B |
| 50 | 685,3/684,3 | 31,5 | A | A | B | C |
| 51 | 641,3/640,3 | 33,9 | A | A | A | A |
| 52 | 685,2/684,3 | 31,5 | A | A | A | B |
| 53 | 657,3/656,3 | 36,7 | A | A | A | B |
| 54 | 701,4/700,3 | 34,4 | A | A | B | D |
| 55 | 657,4/656,3 | 37,1 | A | A | A | B |
| 56 | 701,5/700,3 | 34,9 | A | A | A | C |

**2. Gruppe:**

| # | MS (gefunden/berechnet) | HPLC %AN | Plasmin | PK | Xa | Thrombin |
|---|---|---|---|---|---|---|
| 57 | 708,6/707,38 | 36,5 | A | A | B | D |
| 58 | 752,4/751,4 | 31,9 | B | A | C | D |
| 59 | 608,4/607,3 | 41,0 | B | A | C | D |
| 60 | 666,8/665,3 | 34,4 | B | A | C | D |
| 61 | 566,5/565,3 | 21,5 | B | A | D | D |
| 62 | 694,9/693,4 | 34,6 | A | A | C | C |
| 63 | 623,6/622,3 | 34,8 | A | A | C | C |
| 64 | 681,7/680,3 | 34,7 | A | A | D | D |
| 65 | 667,7/666,3 | 30,1 | B | A | C | D |
| 66 | 681,3/680,8 | 34,4 | A | A | B | D |
| 67 | 667,5/666,8 | 30,1 | A | A | B | D |
| 68 | 567,8/566,3 | 25,4 | B | A | D | D |
| 69 | 625,6/624,3 | 26,1 | B | A | D | D |
| 70 | 611,9/610,3 | 21,0- | B | A | D | D |
| 71 | 623,7/622,3 | 33,9 | A | A | D | C |
| 72 | 567,7/566,3 | 24,6 | B | B | D | D |
| 73 | 637,6/636,3 | 35,1 | B | B | C | B |
| 74 | 581,5/580,3 | 25,2 | B | B | D | D |
| 75 | 636,5/635,3 | 33,2 | B | B | C | C |
| 76 | 580,6/579,3 | 23,1 | B | B | D | D |
| 77 | 650,6/649,3 | 35,0 | A | A | C | B |
| 78 | 594,6/593,3 | 25,1 | B | A | D | D |

**PEGylierte Verbindungen:**

| Nr. | Masse (gefunden/berechnet) | HPLC %AN | Plasmin [Kᵢ] | PK [Kᵢ] | Xa [Kᵢ] | Thrombin [Kᵢ] |
|---|---|---|---|---|---|---|
| 79a | ∼1000 Da | 34,9 | A | A | C | D |
| 79b | ∼2000 Da | 39,4 | A | - | - | - |
| 79c | ∼5000 Da | 43,6 | A | A | B | D |
| 79d | ∼10000 Da | 45,4 | A | A | C | D |
| 80 | ∼10000 Da | 45,8 | A | A | B | D |
| 81 | ∼10000 Da | 46,4 | A | A | C | C |
| 82 | ∼10000 Da | 46,0 | A | A | C | C |
| 83 | ∼10000 Da | 45,2 | A | A | C | C |

### Ergebnisse:

1. Der Kᵢ-Wert der Plasminhemmung betrug generell <100 nM. Insbesondere bei Verbindungen mit cyclischen Strukturen an R₂ und R₃ war der Kᵢ-Wert deutlich geringer als 100 nM und bei Verbindungen mit einem aromatischen Kohlenstoffcyclus an R₂ und R₃ unterhalb von ca. 10 nM. Überraschenderweise gibt es besonders viele erfindungsgemäße Verbindungen mit einem Kᵢ-Wert unterhalb von 5 nM, z. B. die Verbindungen Nr. 1-11, 13, 14, 16-18, 20-33, 35, 38, 39, 46 und 49-56.
2. Der Kᵢ-Wert der Plasmakallikreinhemmung betrug ebenso generell <100 nM. Insbesondere bei Verbindungen mit einem aromatischen Kohlenstoffcyclus an R₂ und R₃ war der Kᵢ-Wert deutlich geringer als 100 nM. Überraschenderweise gibt es besonders viele erfindungsgemäße Verbindungen mit einem Kᵢ-Wert unterhalb von 1 nM, z. B. die Verbindungen Nr. 1-3, 5-6, 8-25, 27, 29, 34-36, 39, 40, 49-57, 59-61, 64, 65 und 68-70.
3. Durch den Einbau von Homotyrosin oder Pyridin und die entsprechenden N-Oxide als Heterozyklen in P3 konnte die Selektivität gegenüber FXa deutlich reduziert werden.
4. Im allgemeinen konnte man eine deutliche Verminderung der Hemmung von Thrombin erreichen, wenn R₁ eine 3-COOH-Gruppe darstellt Eine weitere Verminderung der Hemmung von Thrombin konnte dadurch erreicht werden, dass R₄ ein Fluoratom, insbesondere in ortho-Stellung, darstellt.
5. Als besonders geeignete Verbindung hat sich die Verbindung gemäß Formel (I) mit i = 0 und ohne R₄ mit folgenden Resten erwiesen:

| Verbindung Nr. | R₁ | R₂ | p | R₃ | o |
|---|---|---|---|---|---|
| 3 | 3-COOH | 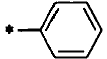 | 3 | 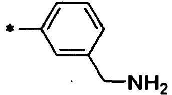 | 1 |

### Literaturstellen

Asghar et al., Biochim Biophys Acta, 438,250-264, 1976
Collen et al., J. Lab. Clin. Med., 99, 76-83, 1982
Dixon et al., Biochem. J., 55, 170-171, 1953
Gustafsson et al., Thromb. Res., 79, 110-118, 1998
Mangano et al., New Engl. J. Med., 354, 353-365, 2006
Muramatu und Fuji, Biochim. Biophys. Acta, 242, 203-208, 1971
Muramatu und Fuji, Biochim. Biophys. Acta, 268, 221-224, 1972
Muramatu et al., Hoppe-Seyler's Z. Physiol. Chem., 363, 203-211, 1982
Ohno et al., Thromb. Res., 19, 579-588, 1980
Okada et al., Chem. Pharm. Bull., 48, 1964-1972, 2000
Okada et al., Biopolymers, 51, 41-50, 1999
Okada et al., Bioorg. Med. Chem. Lett., 10, 2217-2221, 2000
Okamoto et al., Thromb. Res., Suppl. VIII, 131-141, 1988
Sanders und Seto, J. Med. Chem., 42, 2969-2976, 1999
Satoh et al., Chem. Pharm. Bull., 33, 647-654, 1985
Schechter und Berger, Biochem Biophys Res Comm, 27, 157-162, 1967
Schmaier, A.H., Journal of Clinical Investigation, 109, 1007-1009, 2002
Schweinitz et al., J. Biol. Chem., 279, 33613-33622, 2004
Sodha et al., Expert Rev. Cardiovasc. Ther., 4, 151-160, 2006
Stürzebecher et al., J. Med. Chem., 40, 3091-3099, 1997
Tada et al., Biol. Pharm. Bull, 24, 520-524, 2001
Teno et al., Chem. Pharm. Bull., 39, 2930-2936, 1991
Tsuda et al., Chem. Pharm. Bull., 49, 1457-1463, 2001
Xue und Seto, J. Med. Chem., 48, 6908-6917, 2005
US 5,602,253
US 6,472,393
US 2006/0148901

## Patentansprüche

1. Verbindungen der allgemeinen Formel (I) 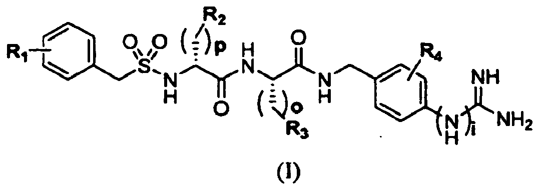 mit
R₁ gegebenenfalls einfach oder mehrfach vorhanden und unabhängig voneinander ein COOR₅-Rest, mit R₅ gleich Wasserstoff oder einer verzweigten oder linearen Niederalkylgruppe mit 1-6 Kohlenstoffatomen, ein verzweigter oder linearer Aminoalkyl-Rest mit 1-6 Kohlenstoffatomen, ein Halogen- oder Pseudohalogen-Rest, oder ein Polyethylenglykol-Rest der Formel (II) oder (III)
CH₃-O-(CH₂-CH₂-O-)ₙ-CH₂-CH₂-(C=O)-NH-CH₂- (II)
CH₃-O-(CH₂-CH₂-O-)ₙ-CH₂-CH₂-NH-(C=O)-CH₂-CH₂-(C=O)-NH-CH₂- (III)
mit n so definiert, dass die Polyethylenglykolreste ein durchschnittliches Molekulargewicht von 10000 Da, 5000 Da, 3400 Da, 2000 Da, 1000 Da oder 750 Da aufweisen;
R₂ ein unsubstituierter oder substituierter, aromatischer oder nichtaromatischer Cyclus oder Bicylus mit 5-13 Kohlenstoffatomen oder aromatischer Heterocyclus mit 4-5 Kohlenstoffatomen und einem Stickstoffatom, Stickstoffoxid, Sauerstoffatom oder Schwefelatom ist, worin die Substitution ein Halogenrest, ein gegebenenfalls mit Fluor substituierter, verzweigter oder linearer Alkyl-Rest mit 1-6 Kohlenstoffatomen, ein gegebenenfalls mit Fluor substituierter, verzweigter oder linearer Alkyloxy-Rest mit 1-6 Kohlenstoffatomen, ein Hydroxy-Rest oder ein Cyano-Rest ist; oder ein Rest der Struktur: 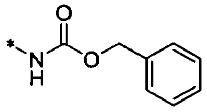 ist;
R₃ ausgewählt ist aus 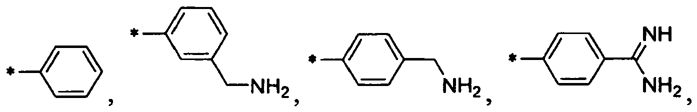 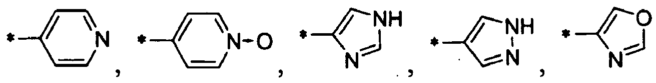 oder 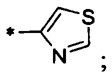 R₄ gegebenenfalls ein einfach oder mehrfach vorhandener Halogen-Rest;
o = 1 oder 2;
p = 0, 1, 2, 3 oder 4; und
i = 0 oder 1;
sowie deren racemische Mischungen und Salze mit organischen oder anorganischen Säuren.

2. Verbindungen nach Anspruch 1, **dadurch gekennzeichnet, dass** in
R₁ der Niederalkylrest Methyl oder Ethyl, vorzugsweise Methyl, der Aminoalkyl-Rest Methyl, und der Halogen- oder Pseudohalogen-Rest Chlor oder eine Cyanogruppe ist; und n eine ganze Zahl von ca. 25 bis ca. 250, vorzugsweise ca. 18, ca. 25, ca. 50, ca. 85, ca. 125 oder ca. 250 ist;
R₂ der aromatische Heterocyclus ein Stickstoffatom oder Stickstoffoxid enthält;
R₄ der Halogen-Rest Fluor ist;
o = 1;
p = 3; und
i = 0.

3. Verbindungen nach Anspruch 1 oder 2, **dadurch gekennzeichnet, dass** R₁ einfach und in meta- oder para-Stellung vorhanden ist, vorzugsweise ist R₁ ein COOH-Rest, insbesondere ist R₁ einfach vorhanden und ausgewählt ist aus Wasserstoff, einer 4-COOH-Gruppe oder insbesondere einer 3-COOH-Gruppe.

4. Verbindungen nach einem der Ansprüche 1-3, **dadurch gekennzeichnet, dass** R₂ ein aromatischer Cyclus oder Bicylus mit 6-13 Kohlenstoffatomen oder Heterocyclus mit 5 Kohlenstoffatomen und einem Stickstoffatom ist.

5. Verbindungen nach einem der Ansprüche 1-3, **dadurch gekennzeichnet, dass** R₂ ein nicht-aromatischer Cyclus mit 6 Kohlenstoffatomen ist.

6. Verbindungen nach einem der Ansprüche 1-5, **dadurch gekennzeichnet, dass** R₃ 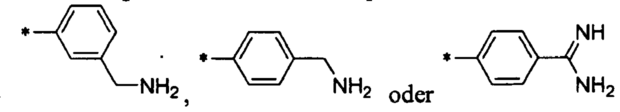 ist.

7. Verbindungen nach einem der Ansprüche 1-6, **dadurch gekennzeichnet, dass** die Salze gebildet sind aus Salzsäure, HBr, Essigsäure, Trifluoressigsäure oder Toluensulfonsäure.

8. Verbindungen nach einem der Ansprüche 1-7, **dadurch gekennzeichnet, dass** R₂ ausgewählt ist aus folgenden Resten:

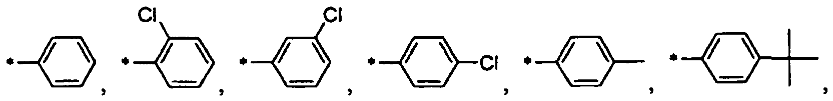

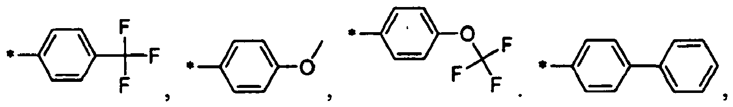

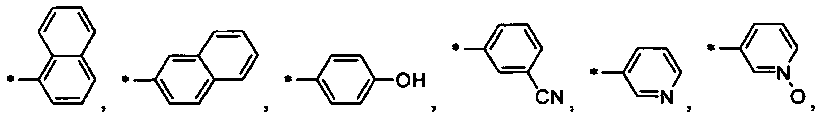

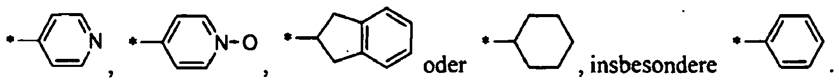

9. Verbindungen nach einem der Ansprüche 1-8, **dadurch gekennzeichnet, dass** R₃
die Struktur 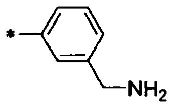 hat.

10. Verbindungen nach einem der Ansprüche 1-9, **dadurch gekennzeichnet, dass** die Verbindung folgendermaßen definiert ist:
| Verbindung Nr. | R₁ | R₂ | p | R₃ | o | i | R₄ |
|---|---|---|---|---|---|---|---|
| 1 | H | 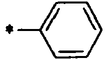 | 3 | 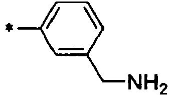 | 1 | 0 | - |
| 2 | 4-COOH | 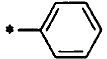 | 3 | 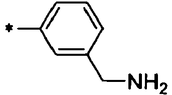 | 1 | 0 | - |
| 3 | 3-COOH | 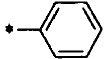 | 3 | 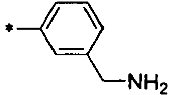 | 1 | 0 | - |
| 4 | 3-COOH | 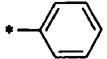 | 3 | 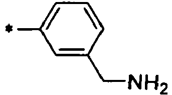 | 1 | 1 | - |
| 5 | 3-COOH | 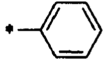 | 3 | 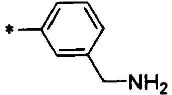 | 1 | 0 | 2-F |
| 6 | H | 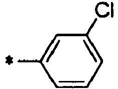 | 3 | 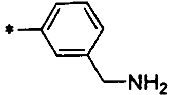 | 1 | 0 | - |
| 7 | H | 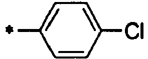 | 3 | 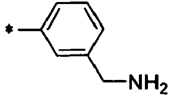 | 1 | 0 | - |
| 8 | H | 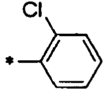 | 3 | 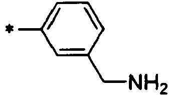 | 1 | 0 | - |
| 9 | H | 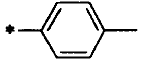 | 3 | 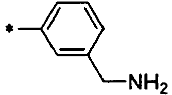 | 1 | 0 | - |
| 10 | H | 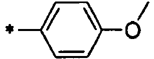 | 3 | 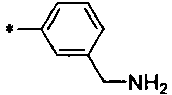 | 1 | 0 | - |
| 11 | H | 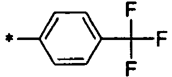 | 3 | 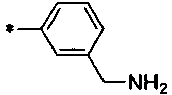 | 1 | 0 | - |
| 12 | H | 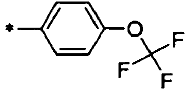 | 3 | 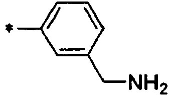 | 1 | 0 | - |
| 13 | H | 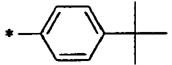 | 1 | 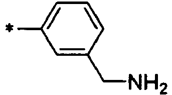 | 1 | 0 | - |
| 14 | H | 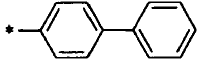 | 1 | 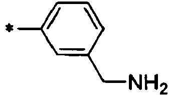 | 1 | 0 | - |
| 15 | H | 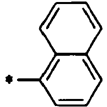 | 1 | 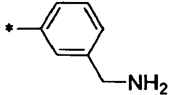 | 1 | 0 | - |
| 16 | 4-COOH | 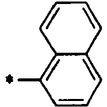 | 1 | 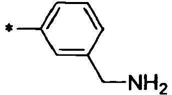 | 1 | 0 | - |
| 17 | H | 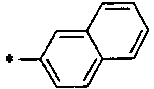 | 1 | 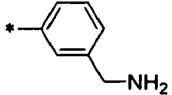 | 1 | 0 | - |
| 18 | 3-COOH | 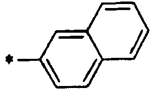 | 1 | 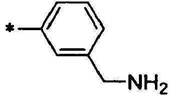 | 1 | 0 | - |
| 19 | 4-COOH | 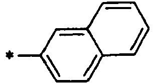 | 1 | 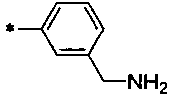 | 1 | 0 | - |
| 20 | H | 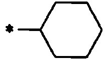 | 1 | 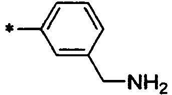 | 1 | 0 | - |
| 21 | H | 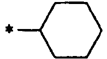 | 3 | 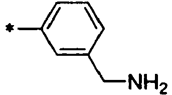 | 1 | 0 | - |
| 22 | H | 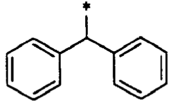 | 0 | 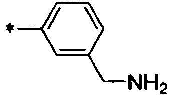 | 1 | 0 | - |
| 23 | 3-COOH | 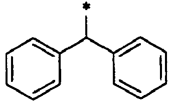 | 0 | 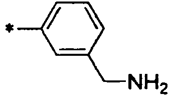 | 1 | 0 | - |
| 24 | H | 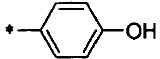 | 2 | 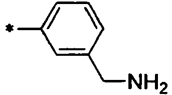 | 1 | 0 | - |
| 25 | 3-COOH | 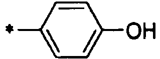 | 2 | 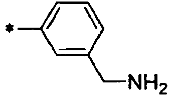 | 1 | 0 | - |
| 26 | H | 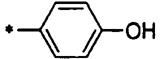 | 2 | 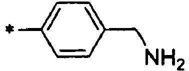 | 2 | 0 | - |
| 27 | H | 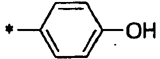 | 2 | 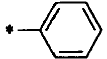 | 2 | 0 | - |
| 28 | H | 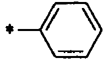 | 3 | 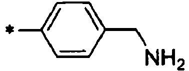 | 1 | 0 | - |
| 29 | 4-COOH | 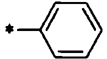 | 3 | 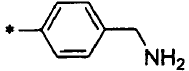 | 1 | 0 | - |
| 30 | 3-COOH | 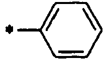 | 3 | 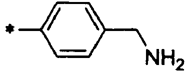 | 1 | 0 | - |
| 31 | H | 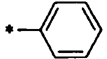 | 3 | 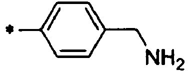 | 2 | 0 | - |
| 32 | H | 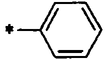 | 3 | 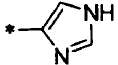 | 1 | 0 | - |
| 33 | 3-COOH | 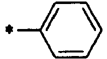 | 3 | 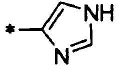 | 1 | 0 | - |
| 34 | H | 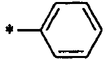 | 3 | 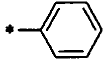 | 2 | 0 | - |
| 35 | 4-COOH | 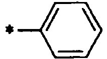 | 3 | 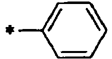 | 2 | 0 | - |
| 36 | 3-COOH | 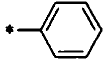 | 3 | 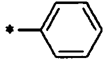 | 2 | 0 | - |
| 37 | H | 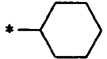 | 1 | 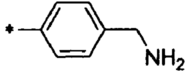 | 2 | 0 | - |
| 38 | H | 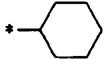 | 1 | 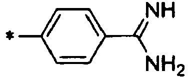 | 2 | 0 | - |
| 39 | H | 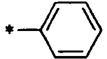 | 3 | 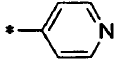 | 2 | 0 | - |
| 40 | 3-COOH | 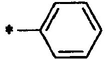 | 3 | 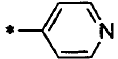 | 2 | 0 | - |
| 41 | H | 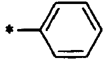 | 3 | 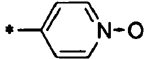 | 2 | 0 | - |
| 42 | 3-COOH | 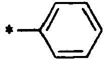 | 3 | 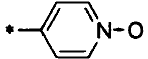 | 2 | 0 | - |
| 43 | H | 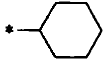 | 1 | 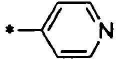 | 2 | 0 | - |
| 44 | H | 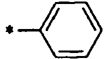 | 2 | 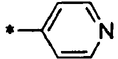 | 2 | 0 | - |
| 45 | H | 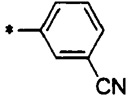 | 1 | 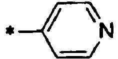 | 2 | 0 | - |
| 46 | H | 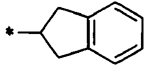 | 0 | 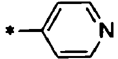 | 2 | 0 | - |
| 47 | H | 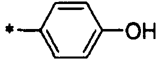 | 2 | 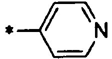 | 2 | 0 | - |
| 49 | H | 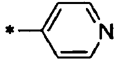 | 3 | 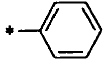 | 2 | 0 | - |
| 50 | 3-COOH | 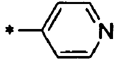 | 3 | 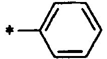 | 2 | 0 | - |
| 51 | H | 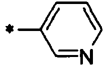 | 3 | 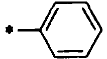 | 2 | 0 | - |
| 52 | 3-COOH | 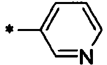 | 3 | 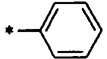 | 2 | 0 | - |
| 53 | H | 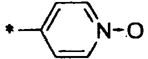 | 3 | 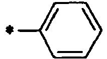 | 2 | 0 | - |
| 54 | 3-COOH | 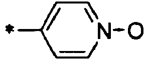 | 3 | 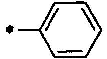 | 2 | 0 | - |
| 55 | H | 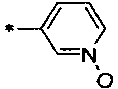 | 3 | 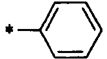 | 2 | 0 | - |
| 56 | 3-COOH | 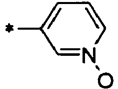 | 3 | 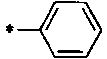 | 2 | 0 | - |

11. Verbindung nach Anspruch 10, **dadurch gekennzeichnet, dass** die Verbindung folgendermaßen definiert ist:
| R₁ | R₂ | p | R₃ | o | i | R₄ |
|---|---|---|---|---|---|---|
| 3-COOH | 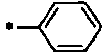 | 3 | 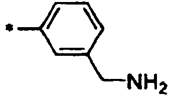 | 1 | 0 | - |

12. Verbindungen der allgemeinen Formel (IV) 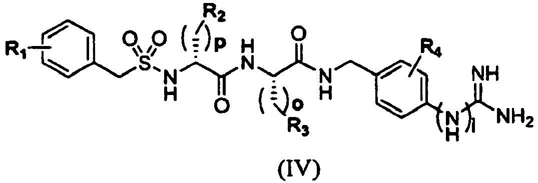 mit
R₁ gegebenenfalls einfach oder mehrfach vorhanden und unabhängig voneinander ein COOR₅-Rest, mit R₅ gleich Wasserstoff oder einer verzweigten oder linearen Niederalkylgruppe mit 1-6 Kohlenstoffatomen, ein verzweigter oder linearer Aminoalkyl-Rest mit 1-6 Kohlenstoffatomen, ein Halogen oder Pseudohalogen-Restoder ein Polyethylenglykol-Rest der Formel (V) oder (VI)
CH₃-O-(CH₂-CH₂-O-)ₙ-CH₂-CH₂-(C=O)-NH-CH₂- (V)
CH₃-O-(CH₂-CH₂-O-)ₙ-CH₂-CH₂-NH-(C=O)-CH₂-CH₂-(C=O)-NH-CH₂- (VI)
mit n so definiert, dass die Polyethylenglykolreste ein durchschnittliches Molekulargewicht von 10000 Da, 5000 Da, 3400 Da, 2000 Da, 1000 Da oder 750 Da aufweisen;
R₂ ein verzweigter oder linearer Alkyloxy-Rest mit 1-6 Kohlenstoffatomen, ein Hydroxy-Rest, Amino-Rest oder ein verzweigter oder linearer Alkyloxycarbonylamido-Rest mit 1-6 Kohlenstoffatomen ist;
R₃ ausgewählt aus folgenden Resten: 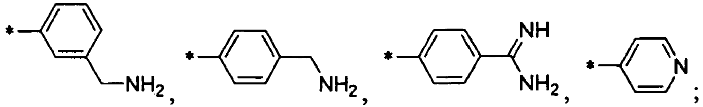 R₄ gegebenenfalls ein einfach oder mehrfach vorhandener Halogen-Rest,
o = 1 oder 2;
p = 1, 2, 3 oder 4,
i = 0 oder 1;
sowie deren racemische Mischungen und Salze mit organischen oder anorganischen Säuren.

13. Verbindungen nach Anspruch 12, **dadurch gekennzeichnet, dass** in
R₁ der Niederalkylrest Methyl oder Ethyl, vorzugsweise Methyl, der Aminoalkyl-Rest Methyl, und der Halogen- oder Pseudohalogen-Rest Chlor oder eine Cyanogruppe ist; und n eine ganze Zahl von ca. 25 bis ca. 250, vorzugsweise ca. 18, ca. 25, ca. 50, ca. 85, ca. 125 oder ca. 250 ist;
R₂ der Alkoxy- und der Alkyloxycarbonylamido-Rest tertiäres Butyl sind;
R₃ 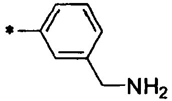 ist;
R₄ der Halogen-Rest Fluor ist;
o = 1;
p = 3; und
i =0.

14. Verbindungen nach Anspruch 13, **dadurch gekennzeichnet, dass** R₁ einfach und in meta- oder para-Stellung vorhanden ist, vorzugsweise ist R₁ Wasserstoff oder ein COOH-Rest, insbesondere ist R₁ einfach vorhanden und ausgewählt ist aus Wasserstoff, einer 4-COOH-Gruppe oder einer 3-COOH-Gruppe.

15. Verbindungen nach einem der Ansprüche 13-14, **dadurch gekennzeichnet, dass** die Salze gebildet sind aus Salzsäure, HBr, Essigsäure, Trifluoressigsäure oder Toluensulfonsäure.

16. Verbindungen nach einem der Ansprüche 13-15, **dadurch gekennzeichnet, dass** die Verbindung folgendermaßen definiert ist:
| Verbindung Nr. | R₁ | R₂ | p | R₃ | o |
|---|---|---|---|---|---|
| 57 | H | 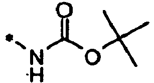 | 4 | 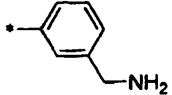 | 1 |
| 58 | 4-COOH | 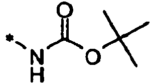 | 4 | 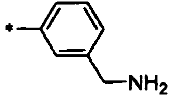 | 1 |
| 59 | H | *-NH₂ | 4 | 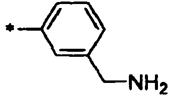 | 1 |
| 60 | H | 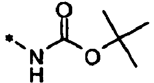 | 1 | 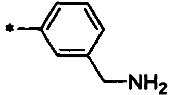 | 1 |
| 61 | H | *-NH₂ | 1 | 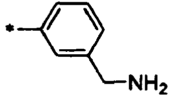 | 1 |
| 62 | H | 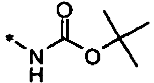 | 4 | 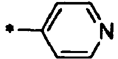 | 2 |
| 63 | H | 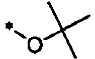 | 1 | 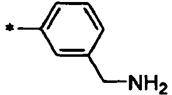 | 1 |
| 64 | 4-COOMe | 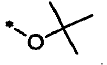 | 1 | 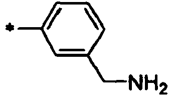 | 1 |
| 65 | 4-COOH | 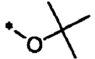 | 1 | 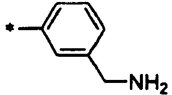 | 1 |
| 66 | 3-COOMe | 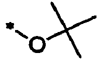 | 1 | 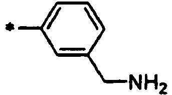 | 1 |
| 67 | 3-COOH | 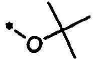 | 1 | 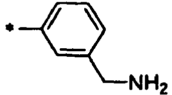 | 1 |
| 68 | H | *-OH | 1 | 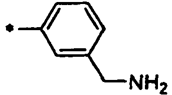 | 1 |
| 69 | 4-COOMe | *-OH | 1 | 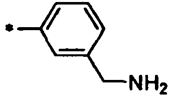 | 1 |
| 70 | 4-COOH | *-OH | 1 | 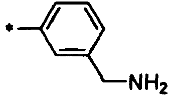 | 1 |
| 71 | H | 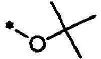 | 1 | 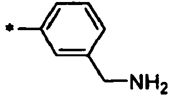 | 1 |
| 72 | H | *-OH | 1 | 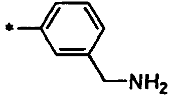 | 1 |
| 73 | H | 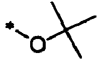 | 1 | 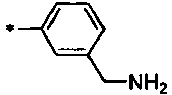 | 2 |
| 74 | H | *-OH | 1 | 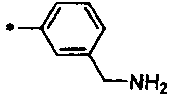 | 2 |
| 75 | H | 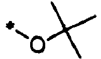 | 1 | 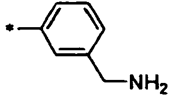 | 1 |
| 76 | H | *-OH | 1 | 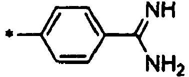 | 1 |
| 77 | H | 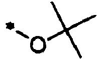 | 1 | 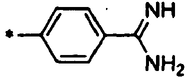 | 2 |
| 78 | H | *-OH | 1 | 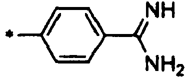 | 2 |

17. Verfahren zur Herstellung einer Verbindung nach einem der Ansprüche 1-16, **dadurch gekennzeichnet, dass** sequentiell die entsprechenden Aminosäuren an ein an der Amidino- oder Guanidinogruppe geschütztes Amidino oder Guanidinobenzylamin angekoppelt werden, wobei die N-terminale Aminosäure entweder den P4-Rest, worin P4 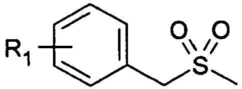 ist, bereits trägt oder dieser anschließend daran gebunden wird, und anschließend die erhaltenen Verbindungen gereinigt und gegebenenfalls PEGyliert werden.

18. Verfahren zur Herstellung einer Verbindung nach einem der Ansprüche 1-16 umfassend folgende Schritte:
(a) Amidierung einer entsprechend Nα-geschützten Aminosäure mit dem Rest R₃, worin R₃ wie in Anspruch 1 definiert ist, mit einem entsprechenden geschütztem Aminomethylbenzamidin oder -guanidin,
(b) Abspaltung der Nα-Schutzgruppe der Aminosäure mit R₃ und Umsetzung des erhaltenen Produktes mit der entsprechenden Benzylsulfonylaminosäure mit den Resten R₁ und R₂, worin R₁ und R₂ wie in Anspruch 1 definiert sind, und Abspaltung restlicher Schutzgruppen zur Verbindung der Formel (I), und nach einer möglichen Reinigung
(c) gegebenenfalls PEGylierung der erhaltenen Verbindung.

19. Arzneimittel enthaltend mindestens eine Verbindung nach einem der Ansprüche 1-16.

20. Arzneimittel nach Anspruch 19 zur Behandlung von Blutverlust, insbesondere bei hyperfibrinolytischen Zuständen, bei Organtransplantationen oder herzchirurgischen Eingriffen vor allem mit cardiopulmonalem Bypass.

21. Fibrinkleber enthaltend mindestens eine Verbindung nach einem der Ansprüche 1-16.

22. Verwendung mindestens einer Verbindung nach einem der Ansprüche 1-16 zur Herstellung eines Arzneimittels nach Anspruch 19 oder 20 oder eines Fibrinklebers nach Anspruch 21.

## Claims

1. Compounds of the general formula (I) 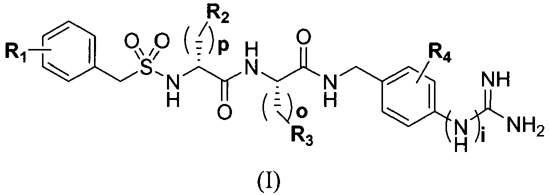 with
R₁ optionally present one or more times and independently of one another a COOR₅ residue, with R₅ equal to hydrogen or a branched or linear lower alkyl group having 1-6 carbon atoms, a branched or linear aminoalkyl residue having 1-6 carbon atoms, a halogen or pseudohalogen residue, or a polyethylene glycol residue of the formula (II) or (III)
CH₃-O-(CH₂-CH₂-O-)ₙ-CH₂-CH₂-(C=O)-NH-CH₂- (II)
CH₃-O-(CH₂-CH₂-O-)ₙ-CH₂-CH₂-NH-(C=O)-CH₂-CH₂-(C=O)-NH-CH₂- (III)
where n is defined such that the polyethylene glycol residues have an average molecular weight of 10,000 Da, 5,000 Da, 3,400 Da, 2,000 Da, 1,000 Da or 750 Da;
R₂ being an unsubstituted or substituted, aromatic or nonaromatic cyclic or bicyclic ring with 5-13 carbon atoms or an aromatic heterocycle having 4-5 carbon atoms and one nitrogen atom, nitrogen oxide, oxygen atom or sulfur atom, wherein the substitution is a halogen residue, a branched or linear alkyl residue having 1-6 carbon atoms optionally substituted with fluorine, a branched or linear alkoxy residue having 1-6 carbon atoms optionally substituted with fluorine, a hydroxy residue or a cyano residue; or a residue of the structure: 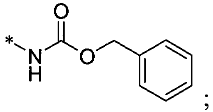 R₃ being selected from 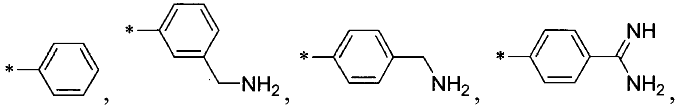 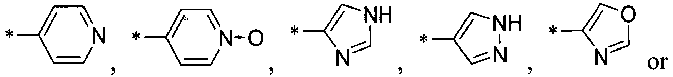 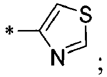 R₄ optionally a halogen residue which is present one or more times;
o= 1 or 2;
p = 0, 1, 2, 3 or 4; and
i= 0 or 1;
as well as the racemic mixtures and salts with organic or inorganic acids thereof.

2. The compounds according to claim 1, **characterized in that** in
R₁ the lower alkyl residue is methyl or ethyl, preferably methyl, the aminoalkyl residue is methyl, and the halogen or pseudohalogen residue is chlorine or a cyano group; and n is an integer from about 25 to about 250, preferably about 18, about 25, about 50, about 85, about 125 or about 250;
R₂ the aromatic heterocycle contains a nitrogen atom or nitrogen oxide;
R₄ the halogen residue is fluorine;
o = 1;
p = 3; and
i = 0.

3. The compounds according to claim 1 or 2, **characterized in that** R₁ is present singly and in the meta- or para- position, R₁ is preferably a COOH residue, in particular R₁ is present singly and is selected from hydrogen, a 4-COOH-group or in particular, a 3-COOH group.

4. The compounds according to any one of claims 1-3, **characterized in that** R₂ is an aromatic ring or bicyclic ring with 6-13 carbon atoms or a heterocycle with 5 carbon atoms and one nitrogen atom.

5. The compounds according to any one of claims 1-3, **characterized in that** R₂ is a nonaromatic ring with 6 carbon atoms.

6. The compounds according to any one of claims 1-5, **characterized in that** R₃ is

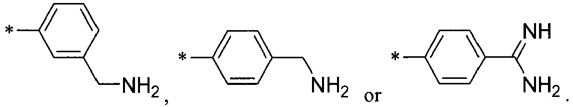

7. The compounds according to any one of claims 1-6, **characterized in that** the salts are formed from hydrochloric acid, HBr, acetic acid, trifluoroacetic acid or toluenesulfonic acid.

8. The compounds according to any of claims 1-7, **characterized in that** R₂ is selected from the following residues:

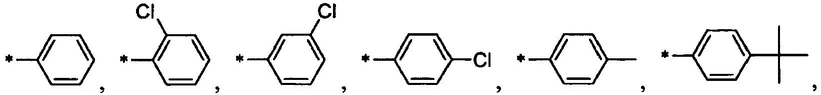

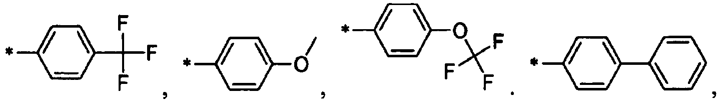

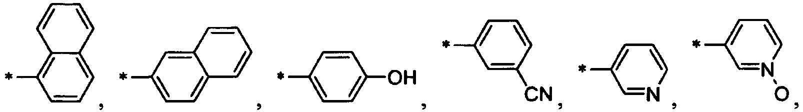

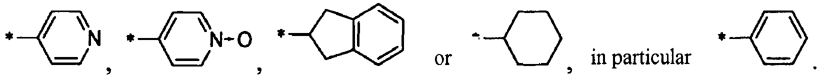

9. The compounds according to any one of claims 1-8, **characterized in that** R₃ has the following structure:

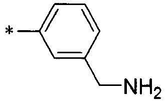

10. The compounds according to any one of claims 1-9, **characterized in that** the compound is defined as follows:
| Compound No. | R₁ | R₂ | p | R₃ | o | i | R₄ |
|---|---|---|---|---|---|---|---|
| 1 | H | 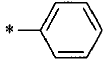 | 3 | 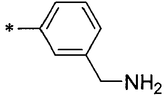 | 1 | 0 | - |
| 2 | 4-COOH | 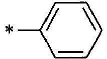 | 3 | 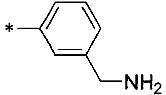 | 1 | 0 | - |
| 3 | 3-COOH | 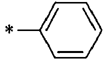 | 3 | 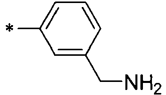 | 1 | 0 | - |
| 4 | 3-COOH | 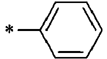 | 3 | 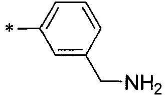 | 1 | 1 | - |
| 5 | 3-COOH | 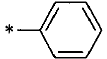 | 3 | 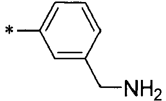 | 1 | 0 | 2-F |
| 6 | H | 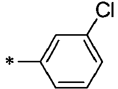 | 3 | 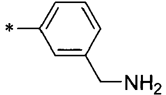 | 1 | 0 | - |
| 7 | H | 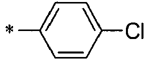 | 3 | 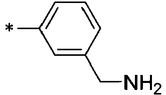 | 1 | 0 | - |
| 8 | H | 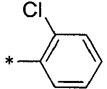 | 3 | 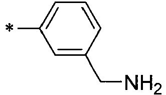 | 1 | 0 | - |
| 9 | H | 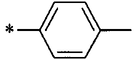 | 3 | 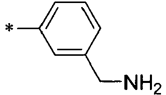 | 1 | 0 | - |
| 10 | H | 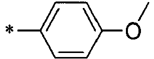 | 3 | 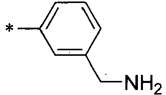 | 1 | 0 | - |
| 11 | H | 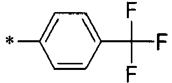 | 3 | 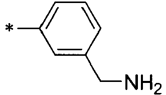 | 1 | 0 | - |
| 12 | H | 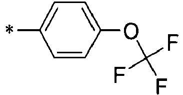 | 3 | 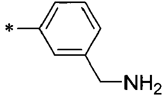 | 1 | 0 | - |
| 13 | H | 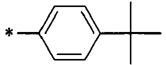 | 1 | 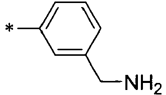 | 1 | 0 | - |
| 14 | H | 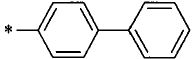 | 1 | 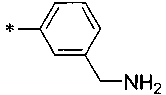 | 1 | 0 | - |
| 15 | H | 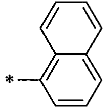 | 1 | 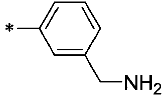 | 1 | 0 | - |
| 16 | 4-COOH | 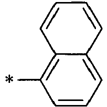 | 1 | 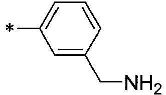 | 1 | 0 | - |
| 17 | H | 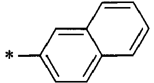 | 1 | 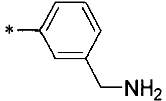 | 1 | 0 | - |
| 18 | 3-COOH | 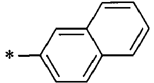 | 1 | 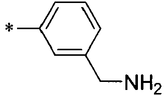 | 1 | 0 | - |
| 19 | 4-COOH | 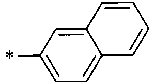 | 1 | 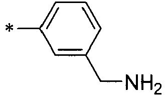 | 1 | 0 | - |
| 20 | H | 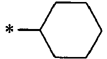 | 1 | 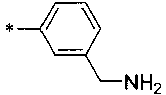 | 1 | 0 | - |
| 21 | H | 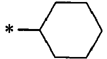 | 3 | 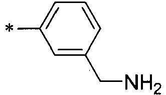 | 1 | 0 | - |
| 22 | H | 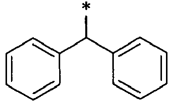 | 0 | 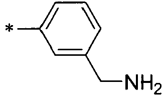 | 1 | 0 | - |
| 23 | 3-COOH | 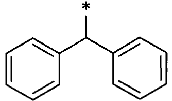 | 0 | 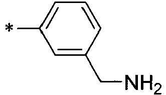 | 1 | 0 | - |
| 24 | H | 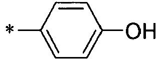 | 2 | 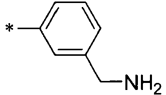 | 1 | 0 | - |
| 25 | 3-COOH | 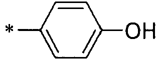 | 2 | 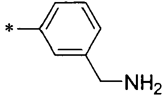 | 1 | 0 | - |
| 26 | H | 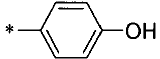 | 2 | 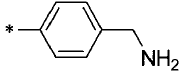 | 2 | 0 | - |
| 27 | H | 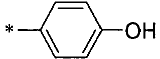 | 2 | 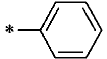 | 2 | 0 | - |
| 28 | H | 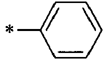 | 3 | 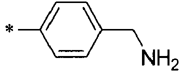 | 1 | 0 | - |
| 29 | 4-COOH | 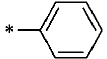 | 3 | 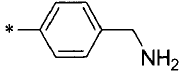 | 1 | 0 | - |
| 30 | 3-COOH | 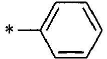 | 3 | 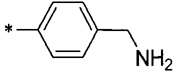 | 1 | 0 | - |
| 31 | H | 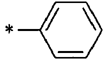 | 3 | 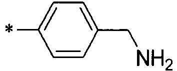 | 2 | 0 | - |
| 32 | H | 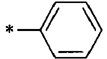 | 3 | 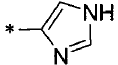 | 1 | 0 | - |
| 33 | 3-COOH | 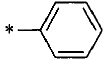 | 3 | 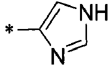 | 1 | 0 | - |
| 34 | H | 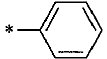 | 3 | 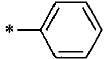 | 2 | 0 | - |
| 35 | 4-COOH | 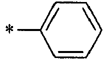 | 3 | 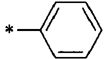 | 2 | 0 | - |
| 36 | 3-COOH | 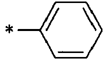 | 3 | 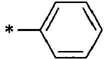 | 2 | 0 | - |
| 37 | H | 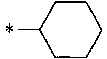 | 1 | 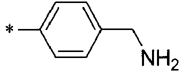 | 2 | 0 | - |
| 38 | H | 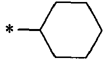 | 1 | 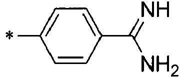 | 2 | 0 | - |
| 39 | H | 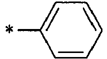 | 3 | 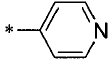 | 2 | 0 | - |
| 40 | 3-COOH | 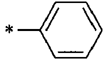 | 3 | 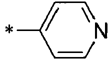 | 2 | 0 | - |
| 41 | H | 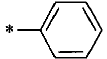 | 3 | 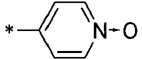 | 2 | 0 | - |
| 42 | 3-COOH | 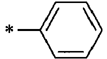 | 3 | 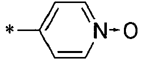 | 2 | 0 | - |
| 43 | H | 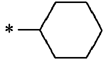 | 1 | 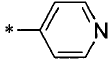 | 2 | 0 | - |
| 44 | H | 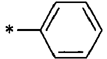 | 2 | 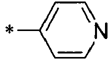 | 2 | 0 | - |
| 45 | H | 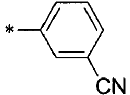 | 1 | 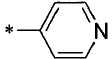 | 2 | 0 | - |
| 46 | H | 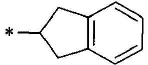 | 0 | 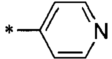 | 2 | 0 | - |
| 47 | H | 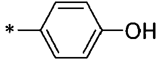 | 2 | 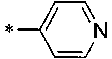 | 2 | 0 | - |
| 49 | H | 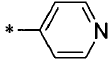 | 3 | 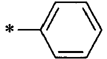 | 2 | 0 | - |
| 50 | 3-COOH | 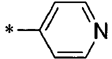 | 3 | 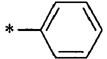 | 2 | 0 | - |
| 51 | H | 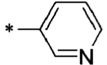 | 3 | 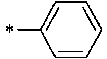 | 2 | 0 | - |
| 52 | 3-COOH | 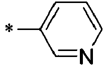 | 3 | 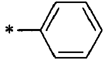 | 2 | 0 | - |
| 53 | H | 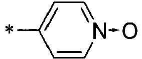 | 3 | 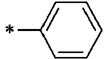 | 2 | 0 | - |
| 54 | 3-COOH | 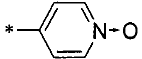 | 3 | 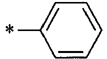 | 2 | 0 | - |
| 55 | H | 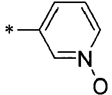 | 3 | 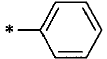 | 2 | 0 | - |
| 56 | 3-COOH | 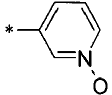 | 3 | 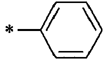 | 2 | 0 | - |

11. The compound according to claim 10, **characterized in that** the compound is defined as follows:
| R₁ | R₂ | p | R₃ | o | i | R₄ |
|---|---|---|---|---|---|---|
| 3-COOH | 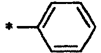 | 3 | 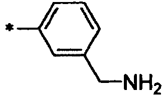 | 1 | 0 | - |

12. Compounds of the general formula (IV) 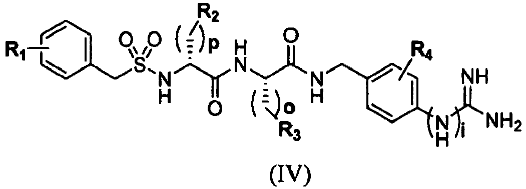 with
R₁ optionally present one or more times and independently of one another a COOR₅ residue, with R₅ equal to hydrogen or a branched or linear lower alkyl group having 1-6 carbon atoms, a branched or linear aminoalkyl residue having 1-6 carbon atoms, a halogen or pseudohalogen residue or a polyethylene glycol residue of the formula (V) or (VI)
CH₃-O-(CH₂-CH₂-O-)ₙ-CH₂-CH₂-(C=O)-NH-CH₂- (V)
CH₃-O-(CH₂-CH₂-O-)ₙ-CH₂-CH₂-NH-(C=O)-CH₂-CH₂-(C=O)-NH-CH₂- (VI)
where n is defined such that the said polyethylene glycol residues have an average molecular weight of 10,000 Da, 5,000 Da, 3,400 Da, 2,000 Da, 1,000 Da or 750 Da;
R₂ being a branched or linear alkyloxy residue with 1-6 carbon atoms, a hydroxy residue, an amino residue or a branched or linear alkyloxycarbonylamido residue with 1-6 carbon atoms;
R₃ being selected from the following residues: 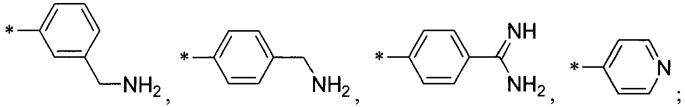 R₄ optionally a halogen residue which is present one or more times,
o = 1 or 2;
p = 1, 2, 3 or 4,
i = 0 or 1;
as well as their racemic mixtures, and salts with organic or inorganic acids.

13. The compounds according to claim 12, **characterized in that** in
R₁ the lower alkyl residue is methyl or ethyl, preferably methyl, the aminoalkyl residue is methyl, and the halogen or pseudohalogen residue is chlorine or a cyano group; and n is an integer from about 25 to about 250, preferably about 18, about 25, about 50, about 85, about 125 or about 250;
R₂ the alkoxy- and the alkyloxycarbonylamido residue are tertiary butyl;
R₃ is 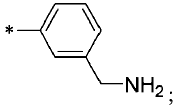 R₄ the halogen residue is fluorine;
o = 1;
p = 3; and
i = 0.

14. The compounds according to claim 13, **characterized in that** R₁ is present singly and in the meta- or para- position, R₁ is preferbably hydrogen or a -COOH residue, R₁ is in particular singly present and is selected from hydrogen, a 4-COOH-group or a 3-COOH group.

15. The compounds according to any one of claims 13-14, **characterized in that** the salts are formed from hydrochloric acid, HBr, acetic acid, trifluoroacetic acid, or toluenesulfonic acid.

16. The compounds according to any one of claims 13-15, **characterized in that** the compound is defined as follows :
| Compound No. | R₁ | R₂ | p | R₃ | o |
|---|---|---|---|---|---|
| 57 | H | 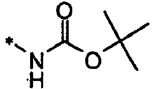 | 4 | 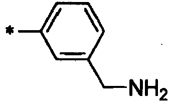 | 1 |
| 58 | 4-COOH | 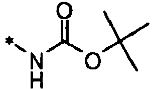 | 4 | 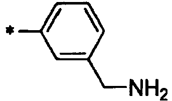 | 1 |
| 59 | H | *-NH₂ | 4 | 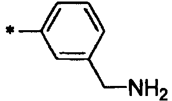 | 1 |
| 60 | H | 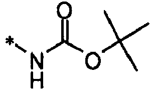 | 1 | 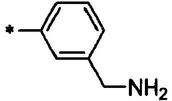 | 1 |
| 61 | H | *-NH₂ | 1 | 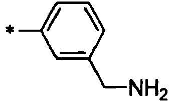 | 1 |
| 62 | H | 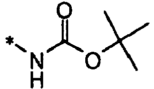 | 4 | 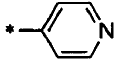 | 2 |
| 63 | H | 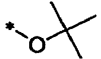 | 1 | 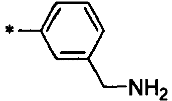 | 1 |
| 64 | 4-COOMe | 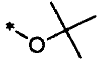 | 1 | 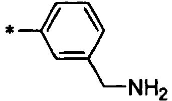 | 1 |
| 65 | 4-COOH | 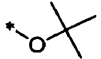 | 1 | 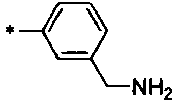 | 1 |
| 66 | 3-COOMe | 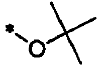 | 1 | 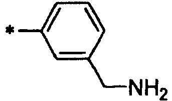 | 1 |
| 67 | 3-COOH | 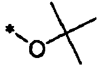 | 1 | 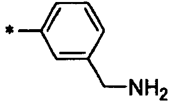 | 1 |
| 68 | H | *-OH | 1 | 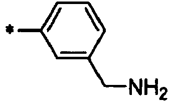 | 1 |
| 69 | 4-COOMe | *-OH | 1 | 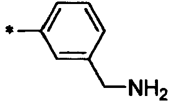 | 1 |
| 70 | 4-COOH | *-OH | 1 | 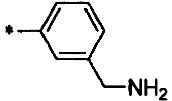 | 1 |
| 71 | H | 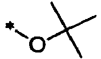 | 1 | 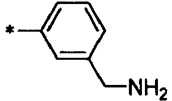 | 1 |
| 72 | H | *-OH | 1 | 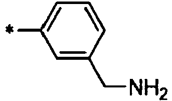 | 1 |
| 73 | H | 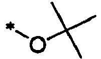 | 1 | 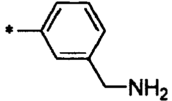 | 2 |
| 74 | H | *-OH | 1 | 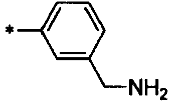 | 2 |
| 75 | H | 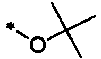 | 1 | 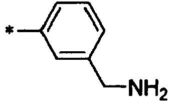 | 1 |
| 76 | H | *-OH | 1 | 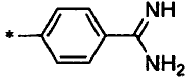 | 1 |
| 77 | H | 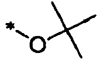 | 1 | 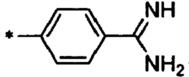 | 2 |
| 78 | H | *-OH | 1 | 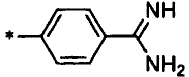 | 2 |

17. A process for the preparation of a compound according to any one of the claims 1-16, **characterized in that** the appropriate amino acids are coupled sequentially to an amidino- or guanidinobenzylamine which is protected at the amidino or guanidino group, wherein the N-terminal amino acid either already carries the P4 residue, wherein P4 is, 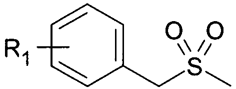 or the latter subsequently being linked thereto, and subsequently the resulting compounds are purified and PEGylated where appropriate.

18. A process for the preparation of a compound according to any one of claims 1-16 comprising the following steps:
(a) amidation of an appropriate Nα-protected amino acid with the residue R₃, wherein R₃ is as defined in claim 1, with an appropriate protected aminomethylbenzamidine or -guanidine,
b) cleavage of the Nα-protecting group of the amino acid of with R₃ and reaction of the obtained product with the appropriate benzylsulfonylamino acid with the residues R₁ and R₂, wherein R₁ and R₂ are as defined in claim 1, and cleavage of remaining protecting groups to give a compound of formula (I), and after possible purification,
c) optional PEGylation of the compound obtained.

19. A medicament containing at least one compound according to any one of claims 1-16.

20. The medicament according to claim 19 for the treatment of blood loss, in particular in hyperfibrinolytic conditions, for organ transplants or heart surgery procedures, in particular cardiopulmonary bypass.

21. Fibrin adhesive containing at least one compound according to any of claims 1-16.

22. Use of at least one compound according to any one of claims 1-16 for the preparation of a medicament according to claim 19 or 20 or of a fibrin adhesive according to claim 21.

## Revendications

1. Composés de formule générale 1 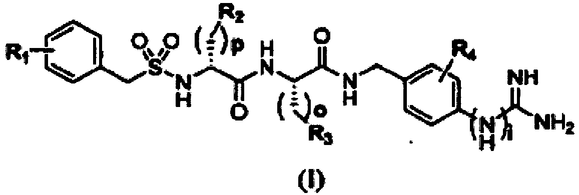 où
R₁ est présent éventuellement une fois ou plusieurs fois et est, indépendamment les uns des autres, est un radical COOR₅, où R₅ est un atome d'hydrogène ou un groupe alkyle inférieur ramifié ou linéaire comportant 1 à 6 atomes de carbone, un radical aminoalkyle ramifié ou linéaire comportant 1 à 6 atomes de carbone, un radical halogène ou pseudo-halogène ou un radical polyéthylène glycol de formule (II) ou (III)
**CH₃-O-(CH₂-CH₂-O-)ₙ-CH₂-CH₂-(C=O)-NH-CH₂-** **(II)**
**CH₃-O-(CH₂-CH₂-O-)ₙ-CH₂-CH₂-NH-(C=O)-CH₂-CH₂-(C=O)-NH-CH₂-** **(III)**
où n est défini de telle sorte que les radicaux polyéthylène glycol ont un poids moléculaire moyen de 10000 Da, 5000 Da, 3400 Da, 2000 Da, 1000 Da ou 750 Da ;
R₂ est un cycle ou bicycle aromatique ou non aromatique, non substitué ou substitué comportant 5 à 13 atomes de carbone ou un hétérocycle aromatique comportant 4 à 5 atomes de carbone et un atome d'azote, un oxyde d'azote, un atome d'oxygène ou un atome de soufre, dans lequel la substitution est un radical halogène, un radical alkyle, ramifié ou linéaire, éventuellement substitué par un atome de fluor, comportant 1 à 6 atomes de carbone, un radical alkyloxy, ramifié ou linéaire, éventuellement substitué par un atome de fluor, comportant 1 à 6 atomes de carbone, un radical hydroxy ou un radical cyano ; ou est un radical de structure : 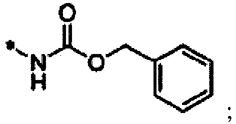 R₃ est choisi parmi 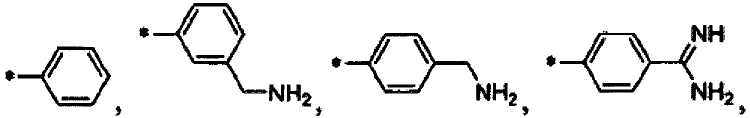 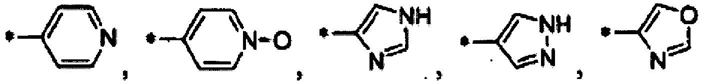 ou 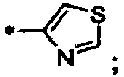 R₄ est éventuellement un radical halogène présent une fois ou plusieurs fois ;
o = 1 ou 2 ;
p = 0, 1, 2, 3 ou 4 ; et
i = 0 ou 1 ;
et leurs mélanges racémiques et leurs sels avec des acides organiques ou inorganiques.

2. Composés selon la revendication 1, **caractérisés en ce que** dans R₁, le radical alkyle inférieur est un méthyle ou éthyle, de préférence méthyle, le radical aminoalkyle est méthyle et le radical halogène ou pseudo-halogène est un atome de chlore ou un groupe cyano ; et n est un nombre entier d'environ 25 à environ 250, de préférence d'environ 18, d'environ 25, d'environ 50, d'environ 85, d'environ 125 ou d'environ 250 ;
R₂, l'hétérocycle aromatique contient un atome d'azote ou un oxyde d'azote ;
R₄, le radical halogène est un atome de fluor ;
o = 1 ;
p=3;et
i = 0.

3. Composés selon la revendication **1** ou **2, caractérisés en ce que** R₁ est présent une fois en position méta ou para, de préférence R₁ est un radical COOH, en particulier R₁ est présent une fois et est choisi parmi l'hydrogène, un groupe 4-COOH ou en particulier un groupe 3-COOH.

4. Composés selon l'une des revendications **1** à **3, caractérisés en ce que** R₂ est un cycle ou bicycle aromatique comportant 6 à 13 atomes de carbone ou un hétérocycle comportant 5 atomes de carbone et un atome d'azote.

5. Composés selon l'une des revendications **1** à **3, caractérisés en ce que** R₂ est un cycle non aromatique comportant 6 atomes de carbone.

6. Composés selon l'une des revendications **1** à **5, caractérisés en ce que** R₃ est

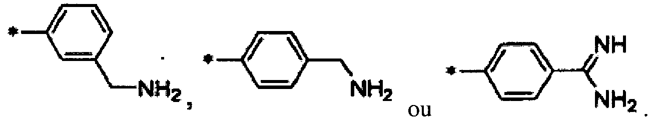

7. Composés selon l'une des revendications **1** à **6, caractérisés en ce que** les sels sont formés à partir de l'acide chlorhydrique, de HBr, de l'acide acétique, de l'acide trifluoroacétique ou de l'acide toluène sulfonique.

8. Composés selon l'une des revendications **1** à **7, caractérisés en ce que** R₂ est choisi parmi les radicaux suivants : 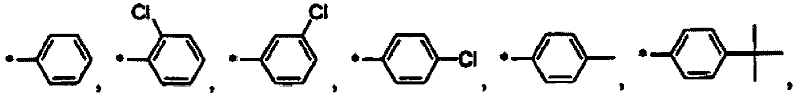 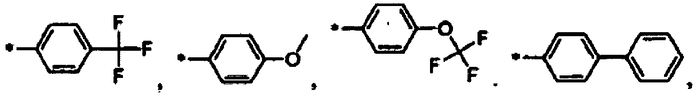 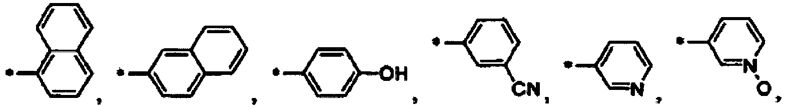 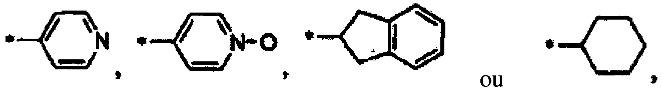 en particulier

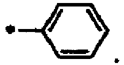

9. Composés selon l'une des revendications **1** à **8, caractérisé en ce que** R₃ a la
structure

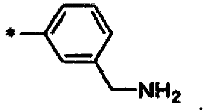

10. Composés selon l'une des revendications **1** à **9, caractérisés en ce que** le composé est défini de la manière suivante :
| Composé n° | R₁ | R₂ | p | R₃ | o | i | R₄ |
|---|---|---|---|---|---|---|---|
| 1 | H | 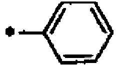 | 3 | 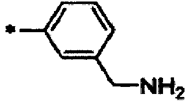 | 1 | 0 | - |
| 2 | 4-COOH | 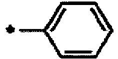 | 3 | 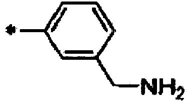 | 1 | 0 | - |
| 3 | 3-COOH | 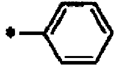 | 3 | 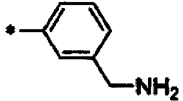 | 1 | 0 | - |
| 4 | 3-COOH | 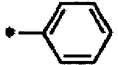 | 3 | 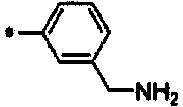 | 1 | 1 | - |
| 5 | 3-COOH | 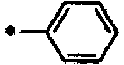 | 3 | 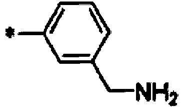 | 1 | 0 | 2-F |
| 6 | H | 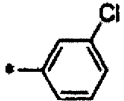 | 3 | 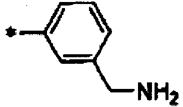 | 1 | 0 | - |
| 7 | H | 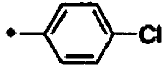 | 3 | 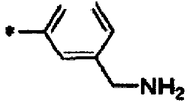 | 1 | 0 | - |
| 8 | H | 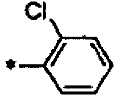 | 3 | 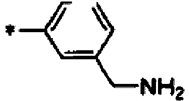 | 1 | 0 | - |
| 9 | H | 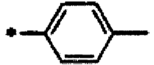 | 3 | 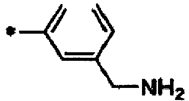 | 1 | 0 | - |
| 10 | H | 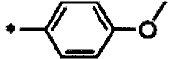 | 3 | 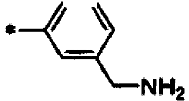 | 1 | 0 | - |
| 11 | H | 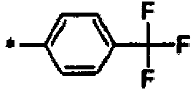 | 3 | 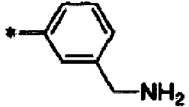 | 1 | 0 | - |
| 12 | H | 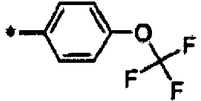 | 3 | 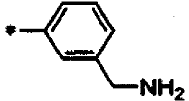 | 1 | 0 | - |
| 13 | H | 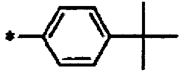 | 1 | 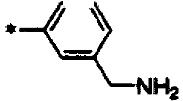 | 1 | 0 | - |
| 14 | H | 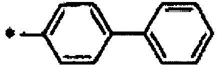 | 1 | 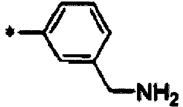 | 1 | 0 | - |
| 15 | H | 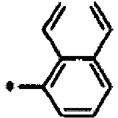 | 1 | 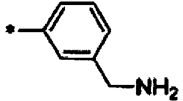 | 1 | 0 | - |
| 16 | 4-COOH | 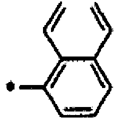 | 1 | 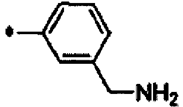 | 1 | 0 | - |
| 17 | H | 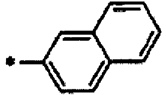 | 1 | 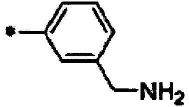 | 1 | 0 | - |
| 18 | 3-COOH | 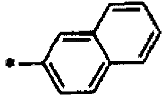 | 1 | 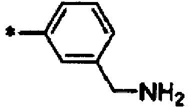 | 1 | 0 | - |
| 19 | 4-COOH | 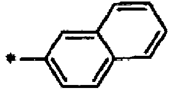 | 1 | 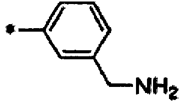 | 1 | 0 | - |
| 20 | H | 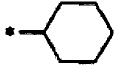 | 1 | 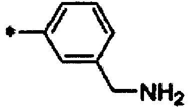 | 1 | 0 | - |
| 21 | H | 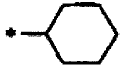 | 3 | 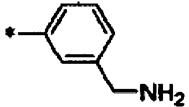 | 1 | 0 | - |
| 22 | H | 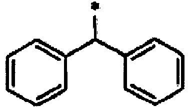 | 0 | 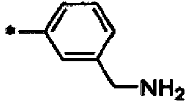 | 1 | 0 | - |
| 23 | 3-COOH | 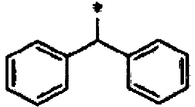 | 0 | 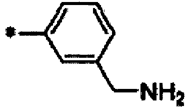 | 1 | 0 | - |
| 24 | H | 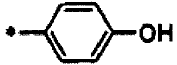 | 2 | 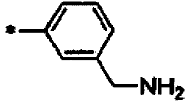 | 1 | 0 | - |
| 25 | 3-COOH | 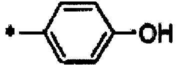 | 2 | 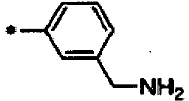 | 1 | 0 | - |
| 26 | H | 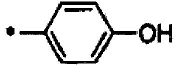 | 2 | 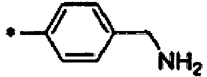 | 2 | 0 | - |
| 27 | H | 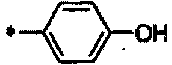 | 2 | 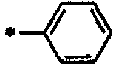 | 2 | 0 | - |
| 28 | H | 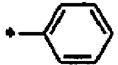 | 3 | 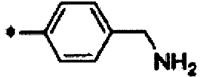 | 1 | 0 | - |
| 29 | 4-COOH | 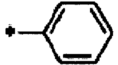 | 3 | 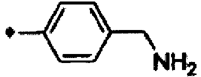 | 1 | 0 | - |
| 30 | 3-COOH | 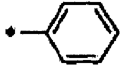 | 3 | 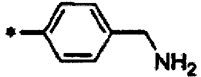 | 1 | 0 | - |
| 31 | H | 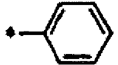 | 3 | 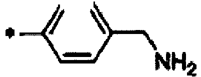 | 2 | 0 | - |
| 32 | H | 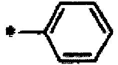 | 3 | 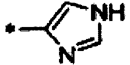 | 1 | 0 | - |
| 33 | 3-COOH | 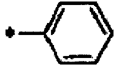 | 3 | 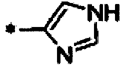 | 1 | 0 | - |
| 34 | H | 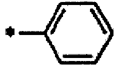 | 3 | 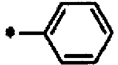 | 2 | 0 | - |
| 35 | 4-COOH | 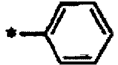 | 3 | 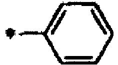 | 2 | 0 | |
| 36 | 3-COOH | 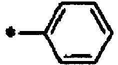 | 3 | 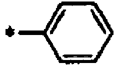 | 2 | 0 | - |
| 37 | H | 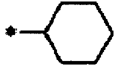 | 1 | 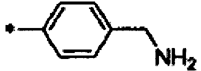 | 2 | 0 | - |
| 38 | H | 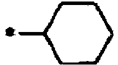 | 1 | 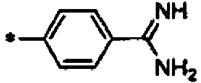 | 2 | 0 | - |
| 39 | H | 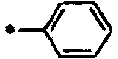 | 3 | 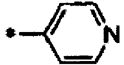 | 2 | 0 | - |
| 40 | 3-COOH | 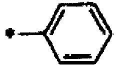 | 3 | 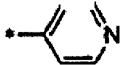 | 2 | 0 | - |
| 41 | H | 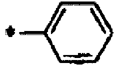 | 3 | 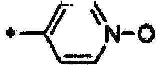 | 2 | 0 | - |
| 42 | 3-COOH | 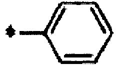 | 3 | 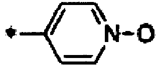 | 2 | 0 | - |
| 43 | H | 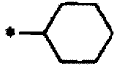 | 1 | 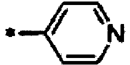 | 2 | 0 | - |
| 44 | H | 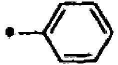 | 2 | 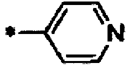 | 2 | 0 | - |
| 45 | H | 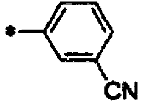 | 1 | 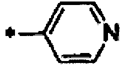 | 2 | 0 | - |
| 46 | H | 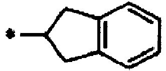 | 0 | 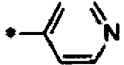 | 2 | 0 | - |
| 47 | H | 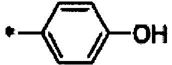 | 2 | 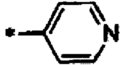 | 2 | 0 | - |
| 49 | H | 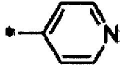 | 3 | 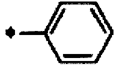 | 2 | 0 | - |
| 50 | 3-COOH | 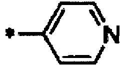 | 3 | 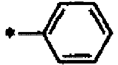 | 2 | 0 | - |
| 51 | H | 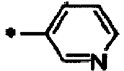 | 3 | 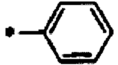 | 2 | 0 | - |
| 52 | 3-COOH | 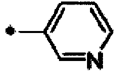 | 3 | 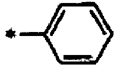 | 2 | 0 | - |
| 53 | H | 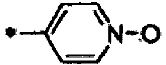 | 3 | 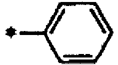 | 2 | 0 | - |
| 54 | 3-COOH | 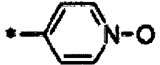 | 3 | 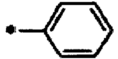 | 2 | 0 | - |
| 55 | H | 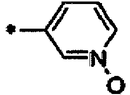 | 3 | 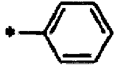 | 2 | 0 | - |
| 56 | 3-COOH | 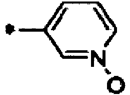 | 3 | 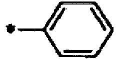 | 2 | 0 | - |

11. Composé selon la revendication **10, caractérisé en ce que** le composé est défini comme suit :
| R₁ | R₂ | p | R₃ | o | i | R₄ |
|---|---|---|---|---|---|---|
| 3-COOH | 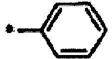 | 3 | 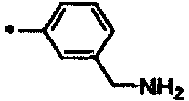 | 1 | 0 | - |

12. Composés de formule générale (IV) 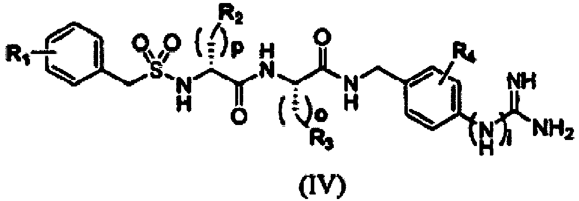 où
R₁ est présent éventuellement une fois ou plusieurs fois et est indépendamment les uns des autres est un radical COOR₅, où R₅ est un atome d'hydrogène ou un groupe alkyle inférieur ramifié ou linéaire comportant 1 à 6 atomes de carbone, un radical aminoalkyle ramifié ou linéaire comportant 1 à 6 atomes de carbone, un radical halogène ou pseudo-halogène ou un radical polyéthylène glycol de formule (V) ou (VI)
**CH₃-O-(CH₂-CH₂-O-)ₙ-CH₂-CH₂-(C=O)-NH-CH₂-** **(V)**
**CH₃-O-(CH₂-CH₂-O-)ₙ-CH₂-CH₂-NH-(C=O)-CH₂-CH₂-(C=O)-NH-CH₂-** **(VI)**
où n est défini de telle sorte que les radicaux polyéthylène glycol ont un poids moléculaire moyen de 10000 Da, 5000 Da, 3400 Da, 2000 Da, 1000 Da ou 750 Da ;
R₂ est un radical alkyloxy ramifié ou linéaire comportant 1 à 6 atomes de carbone, un radical hydroxy, un radical amino ou un radical alkyloxycarbonylamido ramifié ou linéaire comportant 1 à 6 atomes de carbone ;
R₃ est choisi parmi les radicaux suivants : 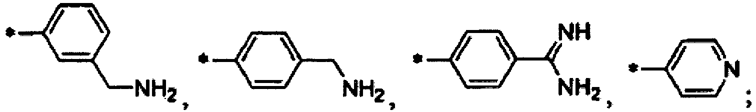 R₄ est éventuellement un radical halogène présent une fois ou plusieurs fois ;
o = 1 ou 2 ;
p = 1, 2, 3 ou 4 ;
i = 0 ou 1 ;
et leurs mélanges racémiques et leurs sels avec des acides organiques ou inorganiques.

13. Composés selon la revendication **12, caractérisés en ce que** dans R₁, le radical alkyle inférieur est un méthyle ou éthyle, de préférence méthyle, le radical aminoalkyle est un méthyle et le radical halogène ou pseudo-halogène est un chlore ou un groupe cyano ; et n est un nombre entier d'environ 25 à environ 250, de préférence d'environ 18, d'environ 25, d'environ 50, d'environ 85, d'environ 125 ou d'environ 250 ; R₂, les radicaux alcoxy et alkyloxycarbonylamido sont un groupe butyle tertiaire ;
R₃ est 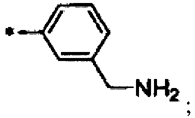 R₄, le radical halogène est un atome de fluor ;
o = 1 ;
p = 3 ; et
i = 0.

14. Composés selon la revendication **13, caractérisés en ce que** R₁ est présent une fois et en position méta ou para, de préférence R₁ est un atome d'hydrogène ou un radical COOH, en particulier R₁ est présent une fois et est choisi parmi l'hydrogène, un groupe 4-COOH ou un groupe 3-COOH.

15. Composés selon l'une des revendications **13** à **14, caractérisés en ce que** les sels sont formés à partir d'acide chlorhydrique, de HBr, d'acide acétique, d'acide trifluoroacétique ou d'acide toluène sulfonique.

16. Composés selon l'une des revendications **13** à **15, caractérisés en ce que** le composé est défini comme suit :
| Composé n° : | R₁ | R₂ | p | R₃ | o |
|---|---|---|---|---|---|
| 57 | H | 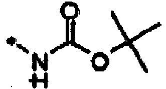 | 4 | 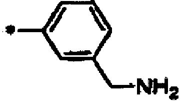 | 1 |
| 58 | 4-COOH | 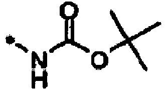 | 4 | 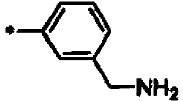 | 1 |
| 59 | H | *-NH₂ | 4 | 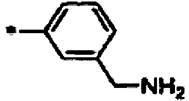 | 1 |
| 60 | H | 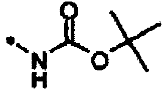 | 1 | 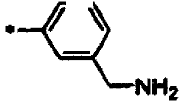 | 1 |
| 61 | H | *-NH₂ | 1 | 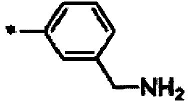 | 1 |
| 62 | H | 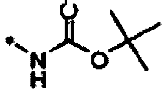 | 4 | 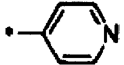 | 2 |
| 63 | H | 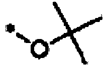 | 1 | 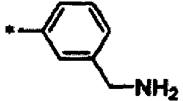 | 1 |
| 64 | 4-COOMe | 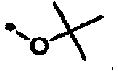 | 1 | 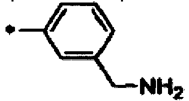 | 1 |
| 65 | 4-COOH | 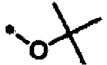 | 1 | 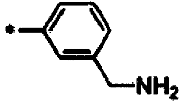 | 1 |
| 66 | 3-COOMe | 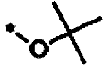 | 1 | 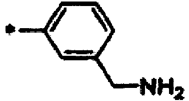 | 1 |
| 67 | 3-COOH | 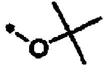 | 1 | 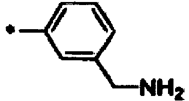 | 1 |
| 68 | H | *-OH | 1 | 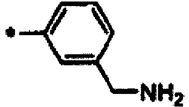 | 1 |
| 69 | 4-COOMe | *-OH | 1 | 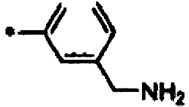 | 1 |
| 70 | 4-COOH | *-OH | 1 | 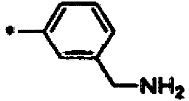 | 1 |
| 71 | H | 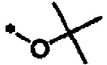 | 1 | 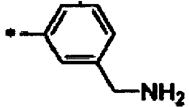 | 1 |
| 72 | H | *-OH | 1 | 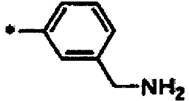 | 1 |
| 73 | H | 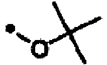 | 1 | 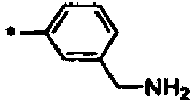 | 2 |
| 74 | H | *-OH | 1 | 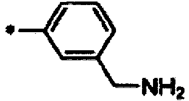 | 2 |
| 75 | H | 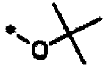 | 1 | 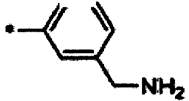 | 1 |
| 76 | H | *-OH | 1 | 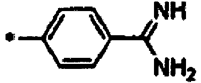 | 1 |
| 77 | H | 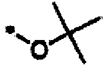 | 1 | 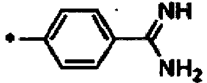 | 2 |
| 78 | H | *-OH | 1 | 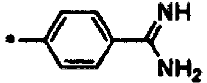 | 2 |

17. Procédé de préparation d'un composé selon l'une des revendications **1** à **16, caractérisé en ce que** les acides aminés correspondants sont séquentiellement couplés à un groupe amidino ou guanidino dont le groupe amidino ou guanidino est protégé sous forme de benzylamine , dans lequel l'acide aminé N-terminal soit est déjà le radical P4,
P4 étant 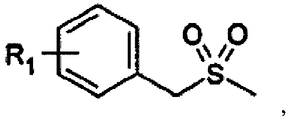 soit est lié ensuite à celui-ci, et ensuite les composés obtenus sont purifiés et éventuellement PEGylés.

18. Procédé de préparation d'un composé selon l'une des revendications **1** à **16,** comprenant les étapes suivantes :
(a) amidation d'un acide aminé protégé par Nα correspondant, avec le radical R₃, où R₃ est tel que défini dans la revendication 1, avec un groupe aminométhylbenzamidine ou aminométhylguanidine protégé correspondant,
(b) clivage du groupe protecteur Nα de l'acide aminé avec R₃ et réaction du produit obtenu avec l'acide benzylsulfonylamino correspondant avec les radicaux R₁ et R₂, où R₁ et R₂ sont tels que définis dans la revendication 1, et clivage des groupes protecteurs restants au composé de formule (I) et après une purification éventuelle,
(c) éventuellement PEGylation du composé obtenu.

19. Médicament contenant au moins un composé selon l'une des revendications **1** à **16.**

20. Médicament selon la revendication **19,** pour le traitement de la perte de sang, en particulier dans les états hyperfibrinolytiques, dans le cas de greffes d'organes ou d'interventions de chirurgie cardiaque, principalement avec pontage cardiopulmonaire.

21. Colle à base de fibrine contenant au moins un composé selon l'une des revendications **1** à **16.**

22. Utilisation d'au moins un composé selon l'une des revendications **1** à **16,** pour la préparation d'un médicament selon la revendication **19** ou **20** ou d'une colle à base de fibrine selon la revendication **21.**
